# FASCICULE DE BREVET EUROPEEN

(11) **EP 1 105 457 B1**
(45) Date de publication et mention de la délivrance du brevet: **06.02.2002**
(21) Numéro de dépôt: 99940269.6
(22) Date de dépôt: 01.09.1999
(51) Int. Cl.: C12M 1/20, C12M 1/34, B01L 3/00

(54) **CARTE DE DENOMBREMENT ET DE CARACTERISATION DE MICRO-ORGANISMES**
TESTKARTE ZUR QUANTIFIZIERUNG UND ZUR NACHWEIS VON MIKROORGANISMEN
CARD FOR COUNTING AND CHARACTERISING MICRO-ORGANISMS

(30) Priorité: 01.09.1998 FR 9811053
(43) Date de publication de la demande: 13.06.2001
(73) Titulaire: BIO MERIEUX, 69280 Marcy l'Etoile (FR)
(72) Inventeur: COLIN, Bruno, F-69280 Marcy l'Etoile (FR); PARIS, Cécile, F-69280 Marcy l'Etoile (FR); SOFIA, Thierry, F-69004 Lyon (FR); TERROT, Claude, F-69007 Lyon (FR)
(74) Mandataire: Guerre, Dominique
(86) Numéro de dépôt international: FR9902082
(87) Numéro de publication internationale: WO0012674

(56) Documents cités:
- EP-A- 0 282 840
- EP-A- 0 550 090
- EP-A- 0 745 856
- DE-U- 29 500 587
- FR-A- 1 598 197
- US-A- 4 018 652
- US-A- 4 077 845
- US-A- 4 318 994
- US-A- 5 149 505

## Description

L'invention concerne une carte d'analyse permettant le dénombrement et la caractérisation des micro-organismes ou de tout autre matériel biologique, tel qu'acides nucléique, anticorps, dans un échantillon biologique. L'invention concerne également un procédé de remplissage d'une telle carte ainsi que l'utilisation de cette carte.

Par le terme « échantillon biologique », il faut comprendre un échantillon liquide issu du traitement physique, par exemple par homogénéisation, ou une dissolution dans un solvant, ou du traitement biologique, comme la digestion enzymatique, de produits solides ou liquides, pouvant être constitués de produits alimentaires, d'eaux, de sang.

*En dehors de la méthode traditionnelle de dénombrement sur boîtes de Pétri, on trouve dans l'état de la technique des méthodes non automatisées pour quantifier les micro-organismes, ces méthodes sont principalement basées sur la détermination du Nombre le Plus Probable (NPP).*

*Le brevet US-A-5, 700, 655 décrit un dispositif de quantification de matériel biologique dans un échantillon liquide. Ce dispositif est constitué d'une plaque en matière plastique obtenue par thermoformage, elle-même constituée de cavités d'analyse de même taille et d'un couvercle permettant de préserver la stérilité. Le procédé consiste à déposer d'abord l'échantillon à analyser au centre de la plaque, puis le milieu réactionnel fourni avec le dispositif. On agite le dispositif, ainsi préparé, de manière à remplir de manière identique toutes les cavités, puis on rejette le mélange réactionnel en excès. Enfin, on incube la plaque pendant une durée suffisante pour mettre en évidence la présence de germes dans un ou plusieurs puits.*

Toutefois, ce dispositif, comme les boîtes de Pétri traditionnelles, ne permet de tester qu'une seule dilution de l'échantillon. Sachant qu'il faut effectuer plusieurs dilutions de l'échantillon afin d'être sûr de pouvoir déterminer la concentration de germes contenus dans ledit échantillon, il sera nécessaire de renouveler cette opération au moins une fois.

De plus, ce dispositif possède un autre inconvénient majeur. Cet inconvénient consiste en la nécessité de rejeter le surplus de mélange réactionnel. En effet, cette opération s'effectuant par inclinaison de la plaque, il est possible que certaines cavités perdent une partie de leur contenu. La quantité de liquide ne sera donc plus identique dans les cavités, et par conséquent la détermination de la quantité de germes sera faussée.

Egalement, le rejet du surplus de mélange réactionnel entraîne la production de déchets potentiellement contaminés, qu'il faut retraiter. De même, ce rejet de surplus contamine l'extérieur du dispositif qui devient alors une source de contamination biologique pour l'opérateur et toutes autres surfaces qui entrent à son contact.

De plus, les cavités étant disposées de façon concentrique, la lecture se fait aussi de façon concentrique, ce qui peut être source d'erreur, et ce d'autant plus que les cavités positives sont souvent dispersées sur la plaque.

Enfin, le dispositif susmentionné conserve l'inconvénient essentiel de la boîte de Pétri, à savoir qu'il faut absolument qu'il soit manipulé avec précaution et horizontalement, le temps que le milieu se gélifie, si cette fonction gélification est prévue, afin d'éviter la fuite d'échantillon ou le passage d'une cavité à l'autre, ce qui induit des artefacts de manipulation.

*Le brevet US-A-5,518,892 montre un autre dispositif de quantification de micro-organismes dans un milieu liquide. Il s'agit d'un sac, fait dans un matériau et obtenu par thermoformage, qui possède une ouverture sur un de ses côtés. Le procédé consiste à former par chauffage, des compartiments ou cavités imperméables sur la face inférieure du sac, permettant la séparation de différentes quantités de l'échantillon. Une fois les puits formés, on prépare le mélange réactionnel en additionnant l'échantillon à analyser et le milieu réactionnel fourni avec le dispositif. On verse alors le mélange réactionnel dans le sac par l'ouverture prévue à cet effet. On vérifie que toutes les cavités sont bien remplies, puis, après scellement thermique, on incube le sac pendant une durée suffisante pour mettre en évidence la présence de micro-organismes dans une ou plusieurs cavités. Les puits peuvent être de même* *taille ou en trois séries de tailles différentes. Il est ainsi possible d'utiliser un seul sac pour simuler un ensemencement selon plusieurs dilutions.*

Cependant, ce système a certains inconvénients. Premièrement, et contrairement à un système de moulage par injection, industriel, automatisé et très précis, ce dispositif ne peut garantir une qualité de moulage optimale permettant d'obtenir des cavités de contenance parfaitement identique, puisqu'il utilise à la base de simples feuilles de matière plastique qui sont mises en forme par thermoformage. Ceci est particulièrement important, lorsqu'on réalise un moulage avec des séries de cavités de différents volumes, notamment lorsque ceux-ci sont de petits volumes. Deuxièmement, on introduit le mélange réactionnel manuellement, la distribution dans les cavités se fait donc de façon aléatoire. Ainsi, on ne peut pas être certain que le volume de mélange réactionnel distribué dans chaque cavité soit le même, même si la taille des cavités est parfaitement identique. Les résultats peuvent donc encore être faussés. Troisièmement, l'isolement des cavités par scellement thermique s'effectue à des températures supérieures à 180°C, ce qui risque d'altérer de manière irréversible (destruction du matériel biologique à analyser et/ou évaporation de tout ou partie du liquide contenant ledit matériel) la fraction d'échantillon contenu dans les cavités. Ceci est d'autant plus sensible que la taille des cavités est petite, et qu'elles contiennent peu de liquide. Cette manipulation peut donc fausser les résultats du dénombrement et de la caractérisation.

Conformément à la présente invention, la carte d'analyse proposée pallie à l'ensemble des inconvénients précédemment cités.

Cette carte permet un remplissage de toutes les cavités, quelle que soit leur taille, qui est proportionnelle d'une cavité à l'autre, et qui n'impose pas de précaution de positionnement lors du remplissage. Par " proportionnelle d'une cavité à l'autre ", il convient de comprendre que lorsque deux cavités sont de mêmes dimensions, la proportion sera alors sensiblement de 1 pour 1, ou lorsque deux cavités sont de dimensions différentes, la proportion est différente de 1 pour 1. Il n'y a d'ailleurs aucun risque de contamination, d'une part, de l'échantillon avec l'extérieur, puisque le liquide reste confiné, et d'autre part, entre les différentes cavités de la carte, puisque en outre une séparation isolante est prévue entre chaque cavité de ladite carte.

Cependant, ce système a certains inconvénients. Premièrement, et contrairement à un système de moulage par injection, industriel, automatisé et très précis, ce dispositif ne peut garantir une qualité de moulage optimale permettant d'obtenir des puits de contenance parfaitement identique, puisqu'il utilise à la base de simples feuilles de plastique qui sont mises en forme par thermoformage. Ceci est particulièrement important, lorsqu'on réalise un moulage avec des séries de puits de différents volumes notamment lorsque ceux-ci sont de petits volumes. Deuxièmement, on introduit le mélange réactionnel manuellement, la distribution dans les puits se fait donc de façon aléatoire. Ainsi, on ne peut pas être certain que le volume de mélange réactionnel distribué dans chaque puits soit le même, même si la taille des puits est parfaitement identique. Les résultats peuvent donc encore être faussés. Troisièmement, l'isolement des puits par scellement thermique s'effectue à des températures supérieures à 180°C, ce qui risque d'altérer de manière irréversible (destruction du matériel biologique à analyser et/ou évaporation de toute ou partie du liquide contenant ledit matériel) la fraction d'échantillon contenus dans les puits. Ceci est d'autant plus sensible que la taille des puits est petite, et qu'ils contiennent peu de liquide. Cette manipulation peut donc fausser les résultats du dénombrement et de la caractérisation.

*Le document US-A-4,077,845 propose une carte d'analyse où chaque cavité est connectée au canal par un siphon qui permet d'introduire des micro-organismes dans les cavités en empêchant sans que cela ne soit une obligation le flux de liquide entre les cavités.*

*Le document DE-U-295 00 587 décrit une carte d'analyse de typage d'un échantillon sanguin. Cette carte est obtenue par assemblage de deux plaques en matière plastique, qui définissent entre elles un réseau de canaux, un orifice d'introduction, un puits de distribution et une pluralité de cavités d'analyse comprenant des anticorps respectivement différents. Les cavités peuvent être traversantes ou à gorge borgne, tandis que un ou deux films transparents recouvrant les faces recto et/ou verso des deux plaques assemblées l'une contre l'autre ne permettent absolument aucun dénombrement de micro-organismes.*

En fait, dans ces deux documents ci-dessus, ils n'essaient pas de répondre au même problème technique que la présente invention, puisque le but recherché avec ces documents est de permettre la croissance de micro-organismes présent dans un échantillon biologique de départ et non pas de réaliser la numération de ces micro-organismes. Il s'agit donc d'un problème qualitatif et non quantitatif.

De plus, si la séparation de l'échantillon entre les puits est évoquée dans le document US-A-4,077,845, il est précisé que la séparation entre les puits n'est pas d'une absolue nécessité. Il ne peut donc y avoir d'application pour la numération de micro-organismes présents dans un échantillon biologique.

*Le document US-A-4,018,652 propose dans une des variantes de réalisation une carte d'analyse, qui comporte des puits de deux volumes différents permettant d'observer la croissance de micro-organismes. Il est possible statistiquement de déterminer le nombre de micro-organismes présents dans l'échantillon initial. La carte, selon une autre variantes, permet d'augmenter encore la portée de cette détermination statistique en proposant trois puits de volumes respectifs petits, moyens et grands.*

*Le document EP-A-0.745.856 décrit sensiblement le même type de carte d'analyse, avec pour unique différence importante la présence de canaux suffisamment longs entre les cavités dont l'objet est de diminuer le risque de contamination croisée entre lesdites cavités.*

*Le document US-A-4,318,994 est également apparenté aux deux documents précédents. La différence essentielle réside dans la forme particulière des canaux d'entrée dans les cavités, qui permettent de prévenir le flux de liquides par capillarité.*

Contrairement à notre invention, ces documents n'utilisent pas de puits qui constitue un moyen de distribution du liquide biologique mais également de stockage d'un fluide inerte permettant d'isoler les cavités entre elles. Ainsi dans le document US-A-4,018,652, les cassettes décrites sont remplies par introduction de l'échantillon liquide dans l'ensemble du réseau de canaux et de puits de la cassette. On peut donc comprendre qu'une fois que l'échantillon a été introduit, il y a une continuité dans la présence du liquide entre tous les puits de la carte et ce quelle que soit leur taille. Une fois que le liquide est présent dans la cassette, les micro-organismes vont donc pouvoir migrer sans aucun problème d'un puits à l'autre ou se développer dans les canaux.

Avec notre invention, chaque puits sert à la distribution de l'échantillon liquide mais pas son stockage, puisque le stockage est associé à un fluide inerte et isolant des aliquotes de liquide présents dans les cavités. Les micro-organismes ne peuvent donc pas se déplacer d'un puits à l'autre. Il n'y a donc pas de risque de contamination entre puits et de possibilité d'erreur de calcul statistique de la concentration initiale en micro-organismes.

De plus, le document EP-A-0.745.856 expose que la présence de bulles peut ruiner l'analyse subséquente, ce qui ne pousse pas l'homme du métier à introduire de l'air ou tout autre fluide inerte dans une telle carte d'analyse. Or dans notre invention, la présence d'un fluide gazeux, par exemple de l'air, est nécessaire pour isoler les différents puits comme cela a été exposé précédemment. Il n'y avait donc aucune évidence à procéder de la sorte.

Conformément à la présente invention, la carte d'analyse proposée pallie à l'ensemble des inconvénients précédemment cités.

Cette carte permet un remplissage de tous les puits, quelle que soit leur taille, qui est proportionnel d'un puits à l'autre, et qui n'impose pas de précaution de positionnement lors du remplissage. Par « proportionnel d'un puits à l'autre », il convient de comprendre que la proportionnalité existe entre deux puits de mêmes dimensions, la proportion sera alors sensiblement de 1 pour 1, ou de puits de dimensions différentes, dans certains cas, les proportions sont différentes de 1 pour 1. Il n'y a d'ailleurs aucun risque de contamination, d'une part, de l'échantillon avec l'extérieur, puisque le liquide reste confiné, et d'autre part, entre les différents puits de la carte, puisqu'une séparation isolante est prévue entre chaque puits de ladite carte.

A cet effet, la présente invention concerne, selon un premier mode de réalisation préféré, une carte d'analyse qui comprend un corps constitué par :
- une zone d'injection dans la carte d'un liquide biologique à analyser,

A cet effet, la présente invention concerne, selon un premier mode de réalisation préféré, une carte d'analyse qui comprend un corps constitué par :
- une zone d'injection dans la carte d'un liquide biologique à analyser,
- un canal principal d'amenée du liquide biologique provenant de la zone d'injection,
- au moins deux canaux secondaires situés dans le prolongement du canal principal, et
- au moins un puits correspondant à chaque canal secondaire,
caractérisée par le fait que chaque puits constitue un moyen de distribution, d'un volume déterminé dudit liquide biologique, dans au moins deux cavités d'analyse terminales, via un canal terminal pour chaque cavité d'analyse, et que le volume total à analyser est inférieur au volume total de toutes les cavités d'analyse.

Selon un deuxième mode de réalisation préféré de l'invention, la carte d'analyse qui comprend un corps constitué par :
- une zone d'injection dans la carte d'un liquide biologique à analyser,
- au moins deux canaux d'amenée du liquide biologique provenant de la zone d'injection, et
- au moins un puits correspondant à chaque canal d'amenée,
   est caractérisée par le fait que chaque puits constitue un moyen de distribution, d'un volume déterminé dudit liquide biologique, dans au moins deux cavités d'analyse terminales, via un canal terminal pour chaque cavité d'analyse, et que le volume à analyser est inférieur au volume total de toutes les cavités d'analyse.

Selon les deux modes de réalisation précédents, la distribution du liquide biologique dans les cavités d'analyse terminales s'effectue en parallèle.

Selon un troisième mode de réalisation préféré de l'invention, la carte d'analyse qui comprend un corps constitué par :
- une zone d'injection dans la carte d'un liquide biologique à analyser,
- un canal principal d'amenée du liquide biologique provenant de la zone d'injection,
- au moins deux canaux secondaires situés dans le prolongement du canal principal, et
- au moins une cavité correspondant à chaque canal secondaire,
caractérisée par le fait que chaque cavité constitue un moyen de réception d'un volume déterminé dudit liquide biologique, et que le volume à analyser introduit dans la carte est supérieur au volume total de toutes les cavités d'analyse.

Selon le mode de réalisation précédent, la distribution du liquide biologique dans les cavités d'analyse s'effectue en série et est suivie d'une purge.Selon le premier mode de réalisation, un puits, faisant office de moyen de distribution principal du liquide biologique, est présent entre le canal principal, d'une part, et les canaux secondaires, d'autre part.

Selon l'un des deux modes de réalisation, les volumes des canaux secondaires sont tous identiques.

Dans un mode préférentiel de réalisation d'un de ces trois modes de réalisation, les cavités d'analyse terminales sont d'au moins deux volumes différents.

Préférentiellement, les cavités d'analyse terminales sont de trois volumes différents.

Le rapport existant entre deux cavités de volumes différents est compris entre 1/5 et 1/20, 1/50 et 1/200 ou 1/500 et 1/2000, et notamment 1/10, 1/100 ou 1/1000.

Les cavités d'un même volume sont disposées géométriquement, par exemple alignées ou concentriques, pour faciliter la lecture optique manuelle et/ou automatique.

La présente invention concerne également le procédé de remplissage d'une carte d'analyse, telle qu'exposée dans les deux premiers modes de réalisation, qui est
caractérisé en ce qu'il consiste à :
- relier la zone d'injection de la carte à un volume d'un échantillon biologique à analyser,
- créer une dépression au sein et/ou au voisinage de ladite carte,
- casser la dépression afin, d'une part, de transférer le liquide biologique dans les cavités d'analyse, et, d'autre part, d'isoler par un fluide inerte les différentes fractions de l'échantillon, issues dudit liquide biologique, qui sont présentes dans chacune desdites cavités d'analyse, et
- analyser le résultat obtenu afin de déterminer la quantité de micro-organismes ou de tout autre matériel biologique, tel qu'acides nucléiques, anticorps, présent dans l'échantillon biologique de départ.

La présente invention concerne également le procédé de remplissage d'une carte d'analyse, telle qu'exposée dans le troisième mode de réalisation, qui est caractérisé en ce qu'il consiste à :
- relier la zone d'injection ou la sortie de la carte à un volume d'un échantillon biologique à analyser,
- créer une dépression ou une surpression au sein et/ou au voisinage de ladite carte, afin de transférer le liquide biologique dans les cavités,
- stopper la dépression ou la surpression,
- purger le canal principal pour isoler par un fluide inerte les différentes fractions de l'échantillon, issues dudit liquide biologique, qui sont présentes dans chacune desdites cavités d'analyse, et
- analyser le résultat obtenu afin de déterminer la quantité de micro-organismes ou de tout autre élément biologique, tel qu'acides nucléiques, anticorps, présent dans l'échantillon biologique de départ.

Selon les deux premiers modes de réalisation de la carte, une fois que la dépression est cassée, le fluide isolant est en contact avec le liquide biologique soit au niveau des canaux secondaires ou des canaux d'amenée soit au niveau des cavités d'analyse.

Préférentiellement, le volume du liquide biologique à analyser est inférieur au volume total de toutes les cavités d'analyse.

Selon le troisième mode de réalisation de la carte, le volume du liquide biologique à analyser est supérieur ou égal au volume total de toutes les cavités d'analyse, de tout le réseau fluidique et du trop-plein.

Préférentiellement, le volume du liquide biologique à analyser est supérieur ou égal au volume total de toutes les cavités d'analyse et de tout le réseau fluidique.

Selon les deux premiers modes de réalisations de la carte, le rapport entre le volume de l'échantillon biologique à analyser et le volume total de toutes les cavités d'analyse est par exemple de 1/10 à 9/10, notamment de 4/10 à 6/10, et préférentiellement de 5/10.

La présente invention concerne enfin une utilisation d'une carte d'analyse, telle que décrite ci-dessus, pour la numération de micro-organismes suivant la technique du nombre le plus probable.

Les figures ci-jointes sont données à titre indicatif et n'ont aucun caractère limitatif sur la portée de la présente invention. Ils constituent plusieurs modes particuliers de réalisation de ladite invention.

La figure 1 représente une vue de dessus d'un premier mode de réalisation d'une carte, selon l'invention, permettant le dénombrement et la caractérisation de micro-organismes dans un échantillon biologique.

La figure 2 représente une vue de dessus d'un deuxième mode de réalisation d'une carte, selon l'invention, permettant le dénombrement et la caractérisation de micro-organismes dans un échantillon biologique.

La figure 3 représente une vue identique mais partielle de la figure 1, mais dans laquelle la carte a été utilisée pour effectuer le dénombrement et la caractérisation d'une population de micro-organismes.

La figure 4 représente une vue identique mais partielle de la figure 1, mais dans laquelle la carte a été utilisée pour effectuer le dénombrement et la caractérisation d'une autre population de micro-organismes.

La figure 5 représente une vue en perspective d'une carte d'analyse, telle que représentée aux figures 1 et 3, associée à un récipient, contenant l'échantillon biologique, par l'intermédiaire d'un tube.

La figure 6 représente une vue en coupe selon A-A de la figure 15.

La figure 7 représente une vue en coupe selon B-B de la figure 5.

La figure 8 représente une vue en coupe identique à celle de la figure 7, mais dans laquelle la carte d'analyse est constituée par un autre mode de réalisation, qui est différent au niveau des cavités.

La figure 9 représente une courbe montrant la variation de la pression au sein d'une cloche à vide où l'ensemble, formé par la carte, l'échantillon, le tube et le tuyau, est placé dans une position permettant par la suite le transfert dudit échantillon dans les cavités de ladite carte.

La figure 10 représente une vue identique mais partielle de la figure 1, mais dans laquelle la carte a été utilisée pour effectuer le dénombrement et la caractérisation d'un autre échantillon contenant des micro-organismes.

La figure 11 représente une vue de dessus d'un troisième mode de réalisation d'une carte, selon l'invention, permettant le dénombrement et la caractérisation de micro-organismes dans un échantillon biologique

La figure 12 représente une vue détaillée d'une cavité d'analyse de taille moyenne.

La figure 13 représente une vue détaillée d'une cavité d'analyse de petite taille.

La figure 14 représente une vue en coupe selon C-C de la figure 11.

La figure 15 représente une vue en coupe selon D-D de la figure 11.

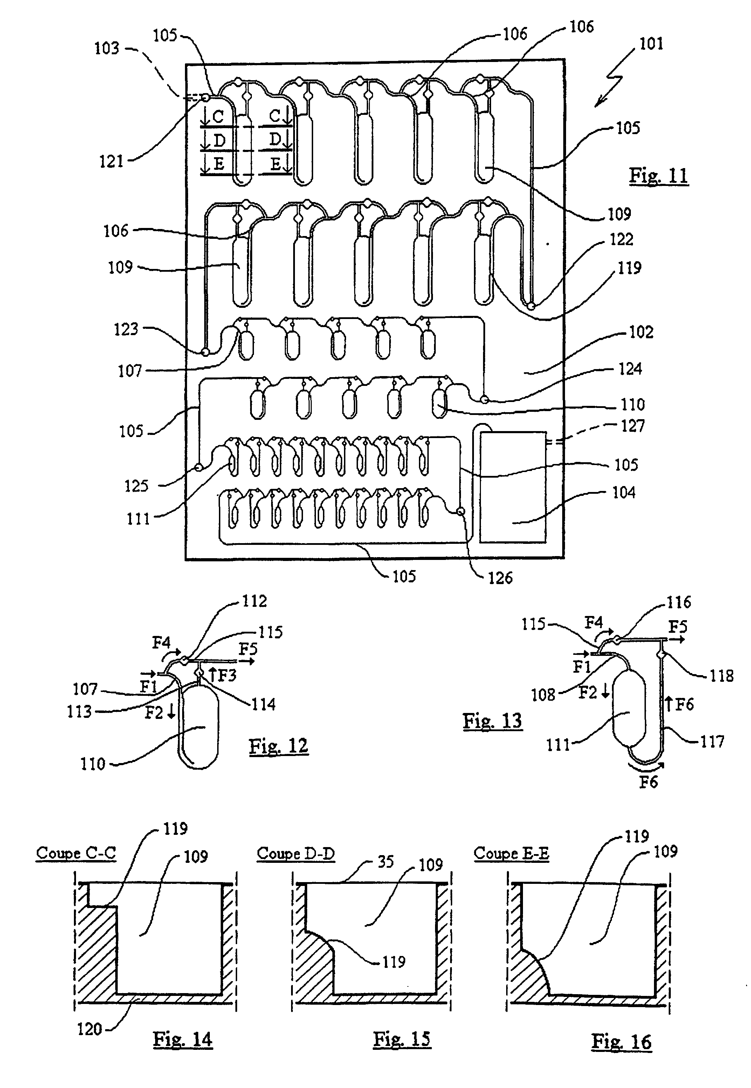

Enfin, la figure 16 représente une vue en coupe selon E-E de la figure 11.

La présente invention concerne essentiellement une carte d'analyse 1 ou 21 ou 101 représentée sur les figures jointes.

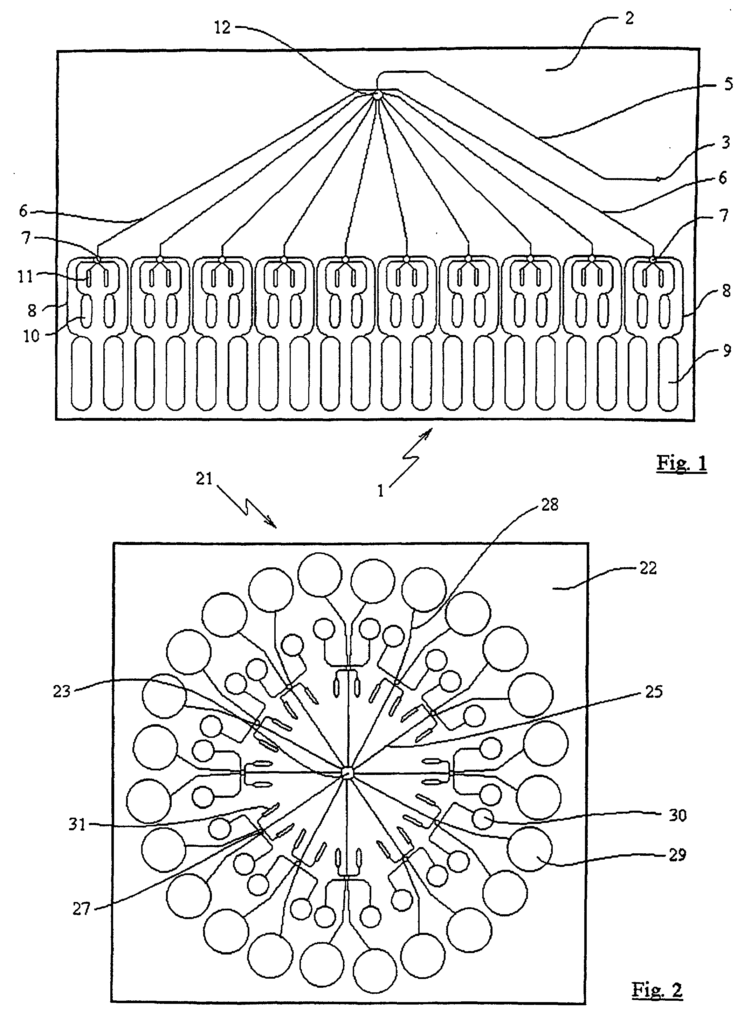

Trois modes particuliers sont représentés, à savoir un premier mode de réalisation d'une carte d'analyse 1 sur les figures 1, 3, 4, 5 et 10, un deuxième mode de réalisation d'une carte d'analyse 21 sur la figure 2 et une troisième mode de réalisation d'une carte d'analyse 101 sur les figures 11 à 16.

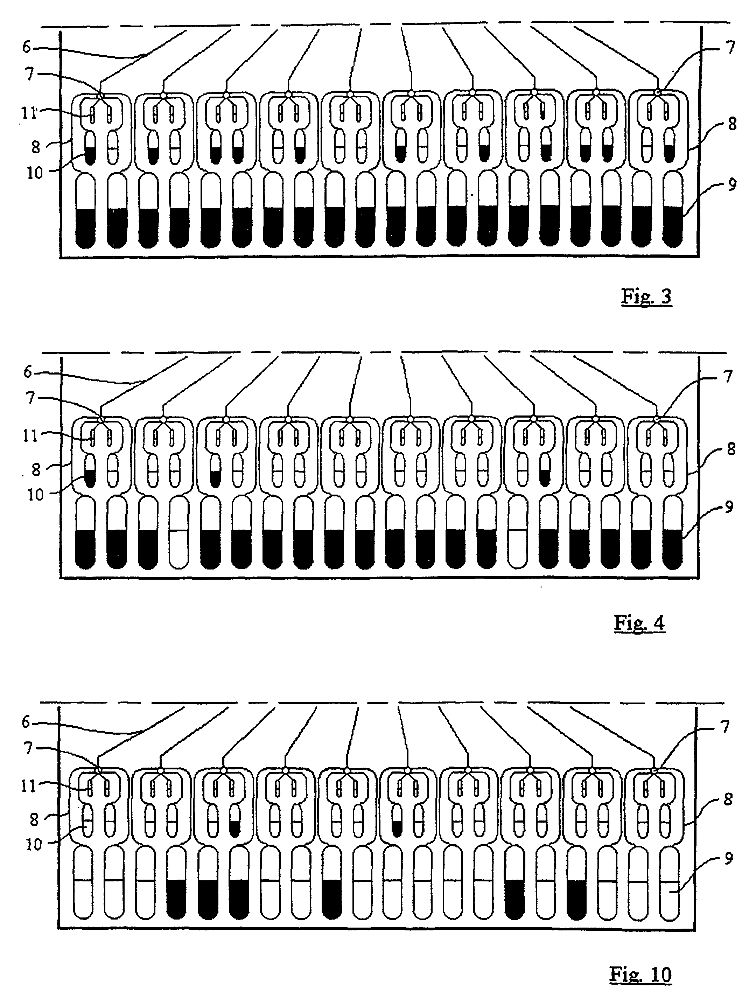

Si on se réfère maintenant aux figures 1 et 3, la carte d'analyse 1 est essentiellement constituée d'un seul élément monobloc constitué par le corps 2 de la carte d'analyse 1. Ce corps 2 comporte une zone d'injection 3 d'un échantillon biologique à analyser 4.

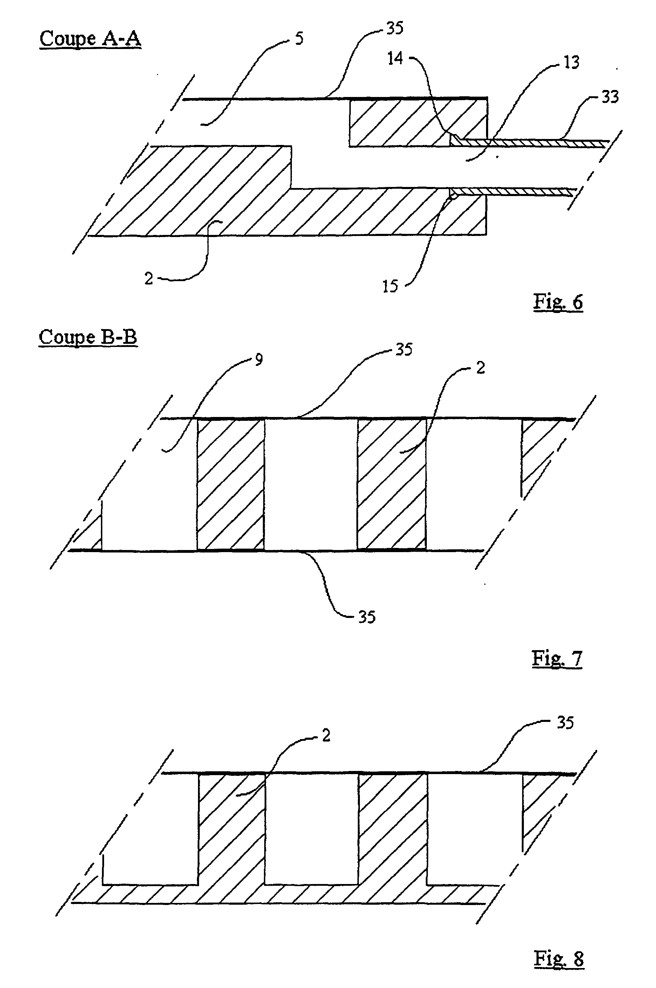

Cette zone d'injection 3 peut être sur n'importe quelle face du corps 2 que ce soit sur la face recto représentée sur la figure 1 ou 3, ou au verso, ou encore sur une paroi latérale. C'est par exemple le cas sur les figures 5 et 6, où la zone d'injection 13 est en position latérale. Sur la figure 5, la carte 1 n'est pas remplie par l'échantillon 4, contenu dans le tube 32, mais est reliée audit échantillon par l'intermédiaire d'un tuyau souple 33. L'ensemble ainsi formé est prêt à être positionné dans une chambre à vide.

La figure 6 représente une coupe selon A-A de la figure 5. Un canal principal d'amenée 5 constitue une chicane pour se diriger au centre du corps 2 de la carte 1, puis se prolonge au sein de ladite carte 1 en direction de l'une de ses surfaces latérales afin de constituer la zone d'injection 3. La liaison existante entre le corps 2 de la carte 1 et le tuyau 33 est assurée par une rainure circulaire 14, située dans le corps 2, et par un épaulement 15 du tuyau 33 coopérant avec la rainure 14.

Bien entendu, pour ce faire, le corps 2 est de forme sensiblement parallélépipédique. Ce corps 2 est donc monobloc et comporte un certain nombre de rainures et de cavités 9, 10 ou 11 situées en son sein. Ces cavités peuvent être constituées par des trous traversants, comme c'est le cas en figure 7, ou par des trous borgnes, comme c'est le cas en figure 8.

L'isolement de ces rainures et de ces trous borgnes par rapport à l'extérieur est effectué par l'application d'un film transparent adhésif 35 qui est collé sur la surface dudit corps 2.

En ce qui concerne les rainures, on note tout d'abord qu'il existe le canal principal d'amenée 5, qui est situé en aval de la zone d'injection 3. En aval de ce canal 5, est présent un puits 12 qui fait office de moyen de distribution principal du liquide biologique 4 à analyser. En fait, le canal principal 5 est situé entre la zone d'injection 3 et le puits 12. De la même façon, il existe des canaux secondaires 6, qui relient ce puits 12 à un ensemble de puits 7, où chaque puits 7 correspond à un canal 6. Ces puits 7 font office de moyens de distribution. Au niveau de ces puits 7, est présent un certain nombre de canaux terminaux 8, chaque canal 8 reliant un puits 7 à une cavité d'analyse terminale 9, 10 ou 11.

En fait, il y a trois types de cavités d'analyse terminales sur ces figures, à savoir, des cavités d'analyse terminales 9 de grande taille, des cavités d'analyse terminales 10 de taille moyenne et enfin des cavités d'analyse terminales 11 de petite taille.

On comprend donc bien que les cavités situées sur le corps 2 de la carte d'analyse 1 sont, d'une part, les rainures qui sont constituées par le canal principal 5, les canaux secondaires 6 et les canaux terminaux 8 et, d'autre part, les trous borgnes qui sont constitués par la zone d'injection 3, le puits 12, les puits 7 et l'ensemble des cavités d'analyse 9, 10 et 11.

Les cavités d'analyse 9, 10 et 11 de tailles différentes permettent de se dispenser de la nécessité d'effectuer des dilutions. Ainsi, en fonction de la quantité de liquide, qui sera présente dans cette cavité d'analyse 9, 10 ou 11, on aura un nombre de micro-organismes qui sera plus ou moins important. En fonction du milieu de culture à ajouter, il sera donc possible de faire croître certains micro-organismes qui en fonction de leur présence ou de leur absence dans chaque cavité 9, 10 ou 11 permettront d'avoir une modification de la densité optique, l'apparition ou la disparition d'une fluorescence ou d'une couleur (effet chromogène). Ceci permettra de détecter la présence ou l'absence de micro-organisme(s) et également d'effectuer une quantification. Des exemples seront exposés plus loin.

Les dimensions de cette carte 1 sont les suivantes selon un mode particulier de réalisation :
- la longueur de la carte 1 est de 135 mm,
- la largeur de ladite carte 1 est de 85,5 mm,
- l'épaisseur de la carte 1 est de 4 mm,
- la largeur de tous les canaux, référencés 5, 6 et 8, est de 0,5 mm,
- la profondeur du canal principal 5 est de 0,5 mm,
- la profondeur des canaux terminaux 6 est variable en fonction de l'éloignement de chaque canal 6 par rapport à un axe virtuel passant par le puits 12, cette profondeur est :
   - de 0,5 mm pour les canaux extérieurs 6,
   - de 0,58 mm pour les canaux centraux et extérieurs 6,
   - de 0,68 mm pour les canaux centraux 6,
   - de 0,79 mm pour les canaux centraux et intérieurs 6,
   - de 0,87 mm pour les canaux intérieurs 6,
- la profondeur des canaux terminaux 8 est de 0,5 mm,
- le diamètre et la profondeur du moyen de distribution principal 12 sont respectivement de 3 mm et de 1 mm,
- le diamètre et la profondeur du moyen de distribution 7 sont respectivement de 2 mm et de 1 mm,
- la position de la zone d'injection 3 est située à 47,5 mm de la base de la carte 1 et à 5 mm au sein de ladite carte 1, il est à noter que cette zone 3 peut être reliée par un canal non représenté sur cette figure 1 au chant de la carte 1 dont le diamètre serait de 2 mm,
- la profondeur des cavités 9 est de 3,56 mm pour une carte pression 500 et est de 2,34 mm pour une carte pression 250, la longueur et la largeur de ces cavités 9 étant constantes et respectivement de 15 mm et de 4,5 mm,
- la profondeur des cavités 10 est de 2 mm pour une carte pression 500 et est de 1,27 mm pour une carte pression 250, la longueur et la largeur de ces cavités 10 étant constantes et respectivement de 6,5 mm et de 2 mm, et
- la profondeur des cavités 11 est de 0,88 mm pour une carte pression 500 et est de 0,53 mm pour une carte pression 250, la longueur et la largeur de ces cavités 11 étant constantes et respectivement de 4 mm et de 0,8 mm.

Par « carte pression 500 », on entend une carte d'analyse 1 subissant une dépression de 500 mbar, c'est-à-dire 500 mbar de pression absolue par rapport au vide absolu. Dans le cas de « carte pression 250 », il s'agit d'une carte d'analyse 1 subissant une dépression de 750 mbar, c'est-à-dire 250 mbar de pression absolue par rapport au vide absolu. Ceci est également vrai pour la carte 21, définie ci-après.

Tous les canaux 5, 6 et 8 sont réalisés par une fraise en forme de boule, alors que les cavités 9, 10 et 11 et les puits 3, 7 et 12 sont réalisés par une fraise cylindrique. Ceci est également vrai pour les autres cartes 21 et 101.

Selon un deuxième mode de réalisation représenté à la figure 2, la carte d'analyse 21 est plus simple puisqu'elle est représentée sous forme étoilée avec une zone d'injection 23 située au centre du corps 22 monobloc de la carte d'analyse 21. En fait, cette zone d'injection 23 correspond au puits 12 de la figure 1, puisque c'est de cette zone 23 que part un certain nombre de canaux d'amenée 25 qui transmettent la diffusion du liquide biologique à analyser 4, non représente sur cette figure 2, en périphérie de la carte d'analyse 21.

Chaque canal d'amenée 25 relie la zone d'injection 23 à un puits 27 faisant office de moyen de distribution. Après ce puits 27, sont présents plusieurs canaux terminaux 28, chaque canal terminal 28 reliant directement un puits 27 à une cavité d'analyse terminale 29, 30 ou 31 qui comporte, à l'instar du mode de réalisation de la figure 1, trois tailles différentes allant de la grande taille référencée 29 à la petite taille référencée 31 en passant par la taille moyenne référencée 30.

Les dimensions de cette carte 21 sont les suivantes selon un mode particulier de réalisation :
- le côté de la carte 21 est de 135 mm,
- l'épaisseur de ladite carte 1 est de 4 mm,
- la largeur et la profondeur de tous les canaux, référencés 25 et 28, sont identiques à 0,5 mm,
- la zone d'injection 23 est un trou qui peut être traversant de 2 mm de diamètre,
- le diamètre et la profondeur du moyen de distribution 27 sont respectivement de 1,5 mm et de 1 mm,
- la profondeur des cavités 29 est de 2,85 mm pour une carte pression 500 et est de 1,88 mm pour une carte pression 250, le diamètre de ces cavités 29 étant de 10 mm,
- la profondeur des cavités 30 est de 1,88 mm pour une carte pression 500 et est de 1,20 mm pour une carte pression 250, le diamètre de ces cavités 30 étant de 4 mm, et
- la profondeur des cavités 31 est de 0,85 mm pour une carte pression 500 et est de 0,49 mm pour une carte pression 250, la longueur et la largeur de ces cavités 31 étant respectivement de 4 mm et de 0,8 mm.

Sur cette figure 2, les cavités d'analyse 29, 30 et 31 sont situées en arc de cercle, ce qui facilite la lecture par un système automatique. Ainsi, il y a mise en rotation de la carte 21 afin que les cavités 29, 30 ou 31 se retrouvent à tour de rôle en face de la tête de lecture, non représentée sur les figures.

Par contre, sur les figures 1 et 3, la lecture est linéaire du fait de l'alignement des différentes cavités d'analyse 9, 10 et 11. De plus, il y a une séparation entre les grandes cavités 9, les cavités moyennes 10 et les petites cavités 11, qui sont superposées les unes au-dessus des autres, ce qui, tout en restant adapté à un système de lecture automatique, facilite la lecture visuelle.

Conformément aux figures 7 et 8, les cavités 9, 10 et 11 peuvent être de différentes formes. Ainsi selon la figure 7, la cavité est traversante, c'est-à-dire que le cloisonnement de chaque cavité nécessite la présence de deux films transparents adhésifs 35. Il est possible également d'avoir un seul film 35 qui prenne en sandwich la carte 1. Cette structure est particulièrement adaptée à une lecture automatisée de la densité optique. Dans le cas de la figure 8, la cavité est constituée par un trou borgne, un seul film 35 est utilisé pour cloisonner l'espace de ladite cavité.

Le film transparent adhésif 35 est d'une utilisation intéressante car, après son perçage ou son retrait, il permet à un opérateur d'avoir un accès direct au contenu des cavités 9 à 11 (Fig. 1) et 29 à 31 (Fig. 2)

Pour simplifier le mode de réalisation des figures 1 et 3, il serait tout à fait possible d'injecter l'échantillon biologique à analyser 4 directement au niveau du puits 12 et ainsi il n'est pas nécessaire d'avoir la zone d'injection 3 et le canal principal d'amenée 5.

On se réfère maintenant à la figure 1, bien que ce qui sera exposé ci-après concerne également l'autre mode de réalisation représenté à la figure 2.

Afin que les volumes distribués dans chaque cavité d'analyse 9, 10 ou 11 soient identiques ou proportionnels pour chaque cavité 9, 10 ou 11 de volumes identiques ou différents, les différents canaux secondaires 6 sont tous d'un volume identique. Ainsi, comme on le remarque bien sur la figure 1, les canaux secondaires 6 situés au centre de la carte d'analyse 1 sont d'une longueur plus faible que ceux situés sur les côtés. Il conviendra donc de jouer sur leur largeur ou sur leur profondeur afin que les volumes soient constants entre tous ces canaux 6. De la même façon, la longueur des canaux terminaux 8 est identique pour chaque cavité d'analyse 9, 10 ou 11 de volume identique.

En fait, il existe également une proportionnalité entre les petites cavités 11 et les grandes cavités 9 ainsi qu'avec les cavités moyennes 10. Ainsi, le rapport existant entre deux cavités de volumes différents, sur les figures représentées, sont de 1 pour 10 entre les cavités 10 et 9 et les cavités 11 et 10 et de 1 pour 100 entre les cavités 11 et 9. Cette proportionnalité peut également se retrouver au niveau des canaux terminaux 8, ces canaux terminaux 8 étant de dimensions différentes en fonction du fait qu'ils alimentent des cavités de grande taille 9, des cavités de taille moyenne 10 ou enfin des cavités de petite taille 11.

S'agissant de la structure des cartes d'analyse 1 et 21, tous les canaux, qu'ils soient principaux 5, d'amenée 25, secondaires 6, terminaux 8 ou 28, ne se croisent pas les uns avec les autres afin d'éviter toute contamination d'une quantité de l'échantillon située dans une cavité d'analyse par rapport à une quantité de l'échantillon située dans une autre cavité d'analyse. Pour ce faire, les points d'intersection de ces différents canaux entre eux, sont toujours réalisés par l'intermédiaire de puits faisant office non seulement de moyen de distribution mais également de volume tampon empêchant toute contamination après l'introduction de l'échantillon 4. Au niveau de ces puits le transfert de liquide à analyser 4 s'effectue en parallèle dans tous les canaux en aval.

Le procédé de remplissage d'une carte d'analyse 1 ou 21, consiste donc à :
- relier la zone d'injection 3 ou 23 de la carte 1 ou 21 à un volume de l'échantillon biologique 4 à analyser,
- générer une dépression au sein et/ou au voisinage de ladite carte 1 ou 21,
- casser progressivement la dépression de sorte que, d'une part, le liquide biologique soit transféré dans les cavités d'analyse 9, 10 et/ou 11 ou bien 29, 30 et/ou 31, et, d'autre part, l'air vienne isoler les différentes fractions de l'échantillon, issues dudit l'échantillon biologique 4, qui sont présents dans chacune desdites cavités d'analyse 9, 10 ou 11 ou bien 29, 30 ou 31.

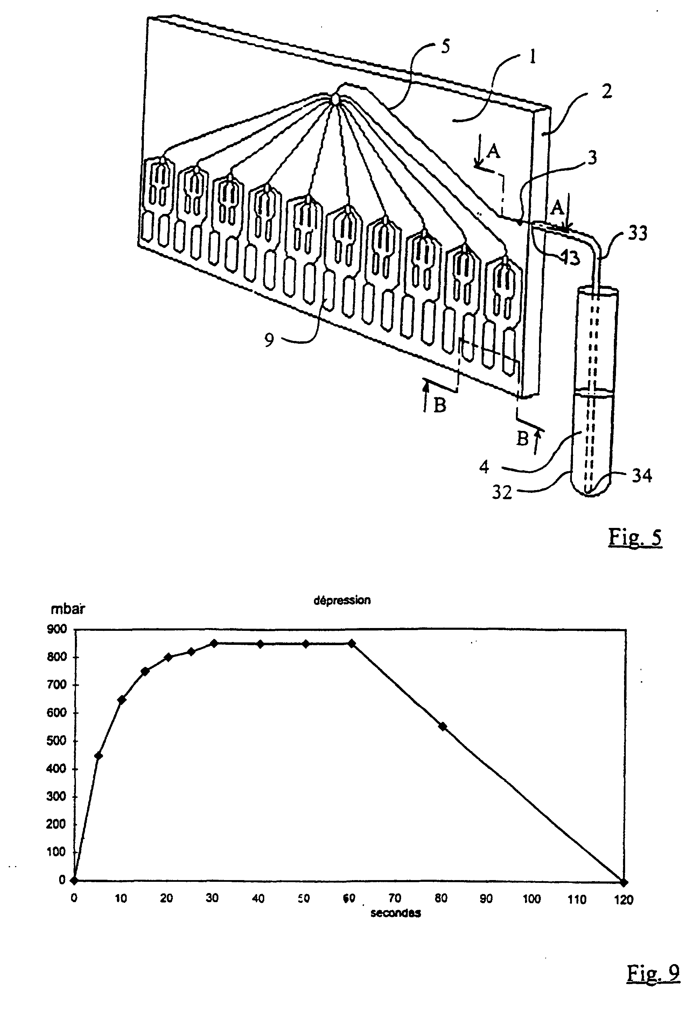

De ce fait, une fois que la dépression est cassée, l'air vient en contact avec les fractions de l'échantillon biologique 4, soit au niveau des canaux terminaux 8 selon les figures 1 et 3, ou des canaux terminaux 28 selon les figures 2 et 4, soit au niveau des cavités d'analyse 9, 10 et 11 (Fig. 1) ou bien 29, 30 et 31 (Fig. 2). Ceci créé une séparation physique entre les bactéries. Bien entendu, ceci n'est réalisable que si le volume de l'échantillon biologique 4 est en rapport avec le volume total des cavités d'analyse ainsi qu'éventuellement du volume des canaux terminaux 8 ou 28.

En lieu et place de l'air, on peut utiliser n'importe quel fluide, gazeux ou liquide, qui pourra assurer cette fonction d'isolement. Il y a lieu de veiller à cette occasion à ce que ledit fluide soit substantiellement inerte par rapport aux composés biologiques contenus dans l'échantillon, et non miscible avec ledit échantillon. Si le fluide est un liquide, comme par exemple de l'huile, il doit être d'une densité plus faible que l'échantillon biologique.

Dans ce cas, la carte 1 doit être maintenue sensiblement en position verticale, comme représenté en figure 5, le fluide isolant, non représenté sur les figures, étant en position supérieure à l'échantillon biologique, contenu dans chaque cavité.

Mais il est possible d'utiliser d'autres techniques pour isoler ou séparer physiquement les cavités 9, 10 et 11 ou 29, 30 et 31. Ainsi, on peut ajouter à l'échantillon biologique 4 et au milieu de culture, permettant la croissance microbienne, un gélifiant, tel que de la peptine. Dans ce cas et lorsque le gélifiant a agi, il n'y a plus de nécessité à maintenir la carte 1 en position sensiblement verticale.

Enfin, l'isolement desdites cavités 9, 10 et 11 ou 29, 30 et 31 peut être réalisée par l'obstruction des canaux terminaux 8. On peut utiliser par exemple une colle comme fluide isolant, ou on peut les obstruer par une compression physique.

Dans un mode particulièrement intéressant de remplissage par le procédé selon l'invention, le volume de l'échantillon biologique 4 à analyser est inférieur au volume total de toutes les cavités d'analyse 9, 10 et 11 (Fig. 1) ou bien 29, 30 et 31 (Fig. 2) Ce rapport entre le volume de liquide biologique 4 et le volume total de toutes les cavités d'analyse 9, 10 et 11 est préférentiellement de un pour deux.

Le remplissage des cavités 9, 10 et 11 ou 29, 30 et 31 s'effectue simultanément, d'ailleurs, à un instant du remplissage donné, le rapport entre le volume de liquide présent dans chaque taille de cavité petite, moyenne ou grande sur le volume total de chaque taille de cavité, respectivement petite, moyenne ou grande, est constant pour la totalité des cavités 9, 10 et 11 ou 29, 30 et 31 de la carte 1 ou 21.

Le troisième mode de réalisation est bien représenté sur les figures 11 à 16. Il se distingue des deux premiers mode de réalisation, d'une part, par sa structure et, d'autre part, par son fonctionnement. Ainsi la structure particulière de ce mode de réalisation permet un transfert du liquide à analyser 4 qui s'effectue en série et non plus en parallèle comme c'était le cas avec les deux premiers modes de réalisation.

La figure 11 permet de bien comprendre le fonctionnement associé à cette structure. La carte d'analyse 101 comporte, à l'instar des précédentes 1 et 21, un corps 102, équipé latéralement d'une zone d'injection 103. Selon un mode particulier de réalisation, cette zone 103 se trouve dans le prolongement du canal principal d'amenée 105, qui parcourt toute la carte 101 avant d'aboutir à un réservoir faisant office de trop-plein 104, lui-même 104 relié à l'extérieur par une sortie 127. On remarque que l'ensemble des cavités d'analyse 109, 110 et 111 est en dérivation par rapport au canal principal 105. Chacune de ces dérivations est référencée 115 au niveau desdites cavités 109, 110 et 111, et ce même si elles sont parties intégrantes de ce canal principal 105. La structure de la carte 101 et de ses constituants influe le liquide 4 dans son mouvement afin que le remplissage des cavités 109, 110 et 111 soit réalisé, mais en isolant les aliquotes présents dans chacune desdites cavités 109, 110 et 111. Une telle structure et son fonctionnement ont déjà fait l'objet de travaux par la demanderesse, ce qui a abouti au dépôt d'une demande de brevet FR98/03033 déposée le 9 mars 1999.

Cette carte 101 comporte toujours dix cavités d'analyse terminales de grande taille 109, dix cavités d'analyse terminales de taille moyenne 110 et dix cavités d'analyse terminales de petite taille 111. Pour une raison d'encombrement, chaque groupe de cavités de taille identique 109, 110 ou 111 est réparti sur deux lignes, ce qui facilite la lecture visuelle de ladite carte 101. Les cavités de grande taille 109 et les cavités de taille moyenne 110 sont de structure quasiment identique et utilisent essentiellement la gravité (au niveau des cavités 109 et 110) associée soit à une dépression appliquée à la sortie 127 soit une surpression appliquée à l'entrée 103 (au niveau du canal principal 105) pour diriger l'échantillon 4, alors que les cavités de petite taille 111 sont d'une structure différente et utilisent essentiellement la capillarité pour leur remplissage par ledit échantillon 4. La dépression appliquée à la sortie 127 ou la surpression appliquée à l'entrée 103 peuvent être comprises entre plus ou moins 5 à 20 mbar et préférentiellement de 10 mbar.

Si l'on se réfère à la figure 11, les cavités de taille moyenne 110, et donc également les cavités de grande taille 109, comportent une intersection entre le canal principal 105 et les canaux secondaires 106, et donc 107. Chaque canal secondaire 106 et 107 aboutit en position supérieure et latérale de la cavité 109 ou 110 concernée. L'extrémité supérieure et centrale de ladite cavité 109 ou 110 la relie au canal principal d'amenée 105, canal 105 qui se dirige vers la cavité 109, 110 ou 111 suivante, par une dérivation supérieure 113. En amont de cette liaison canal principal 105 - dérivation supérieure 113, sont présents deux puits 112 et 114. Le canal principal 105 comporte, au niveau d'une dérivation de contournement 115 de chaque cavité 109 et 110, un puits 112, qui retarde le passage de l'échantillon 4, la direction préférentielle étant la direction du canal secondaire 106 ou 107, par gravité. La dérivation supérieure 113 comporte également un puits 114, qui empêche le passage de l'échantillon 4, la direction préférentielle étant l'écoulement du liquide 4 vers d'autres cavités via le canal principal 105, par aspiration.

Si l'on se réfère maintenant à la figure 13, les cavités de petite taille 111, comportent une intersection entre le canal principal 105 et les canaux secondaires 108. Chaque canal secondaire 108 aboutit en position supérieure et centrale de la cavité 111 concernée. L'extrémité inférieure et centrale de ladite cavité 111 la relie au canal principal d'amenée 105, qui se dirige vers la cavité 111 suivante ou la sortie 127, par une dérivation inférieure 117. En amont de cette liaison canal principal 105 - dérivation inférieure 117, sont présents deux puits 116 et 118. Le puits 116 est présent en amont sur le canal 105 et le puits 118 est présent en amont sur la dérivation 117. Le canal principal 105 comporte, au niveau de chaque cavité 111, un puits 116, qui retarde le passage de l'échantillon 4, la direction préférentielle étant la direction du canal secondaire 108, par capillarité. La dérivation inférieure 117 comporte également un puits 118, qui empêche le passage de l'échantillon 4, la direction préférentielle étant l'écoulement du liquide 4 vers d'autres cavités, via le canal principal 105, par aspiration.

Le canal principal 105 est référencé 115 au niveau des :
- cavités 109 entre la liaison canal principal 105 - canal secondaire 106 et la liaison canal principal 105 - dérivation supérieure 113,
- cavités 110 entre la liaison canal principal 105 - canal secondaire 107 et la liaison canal principal 105 - dérivation supérieure 113,
- cavités 111 entre la liaison canal principal 105 - canal secondaire 108 et la liaison canal principal 105 - dérivation inférieure 117.

On remarque également la présence de différents puits ralentisseurs 121, 122, 123, 124, 125 et 126, représentés en figure 11. Ces puits ralentisseurs 121, 122, 123, 124, 125 et 126 permettent un éventuel contrôle du mouvement du liquide à analyser 4, si l'on souhaite ne remplir que toute ou partie des cavités 109 ou 109 et 110 ou 109, 110 et 111. Bien que cela ne soit pas représenté sur la figure 11, il est possible d'avoir deux vannes en plus, implantées sur le canal principal 105, avant la première cavité 109 et après la dernière cavité 111, ceci afin de diminuer considérablement les risques de contamination interne de la carte 101. L'une des vannes pourrait être implantée au niveau du puits ralentisseur 121 et l'autre avant le trop-plein 104. De telles vannes sont déjà décrites dans les deux demandes de brevet FR98/11383 et FR99/07689 déposées par la demanderesse en dates respectivement du 8 septembre 1998 et du 14 juin 1999.

Les figures 12 et 13 montrent plus en détail le mouvement des fluides (liquide 4 et air) contenus dans la carte 101.

Selon la figure 12, le liquide 4 arrive par le canal principal 105 selon F1 et va s'écouler, préférentiellement selon F2, dans la cavité 110 via le canal secondaire 106. La cavité 110 va alors se remplir jusqu'à ce que le liquide 4 vienne au contact de l'ouverture inférieure de la dérivation supérieure 113. A ce niveau, seul l'air est chassé selon F3 vers le canal principal 105. Le liquide 4 va ensuite prendre la direction de la dérivation de contournement 115 selon F4, pour sortir selon F5 en direction d'une autre cavité 110 ou 111. La dérivation de contournement 115 comporte un puits 112 qui créé une perte de charge à ce niveau ce qui entraîne le liquide à choisir le cheminement le plus aisé, à savoir le canal secondaire 107 et retarde son passage à ce niveau. La dérivation supérieure 113 comporte un puits 114 qui créé également une perte de charge à ce niveau et empêche le passage dudit liquide 4, ce qui pousse le liquide à choisir le cheminement le plus aisé, à savoir la dérivation de contournement 115.

Selon la figure 13, le liquide 4 arrive par le canal principal 105 selon F1 et va s'écouler, préférentiellement selon F2, dans la cavité 111 via le canal secondaire 108. La cavité 111 va alors se remplir complètement, puis le liquide 4 va commencer à s'écouler selon F6 dans une dérivation inférieure 117. Néanmoins, la perte de charge étant très importante à ce niveau, le liquide 4 va ensuite prendre la direction de la dérivation de contournement 115 selon F4, pour sortir selon F5 en direction d'une autre cavité 111 ou du trop-plein 104 et ensuite de la sortie 127. La perte de charge est d'autant plus forte qu'il y a, sur cette dérivation 117, un puits 118 qui empêche le retour dudit liquide 4 dans le canal principal 105. A ce niveau, l'air est donc également chassé selon F6 vers ledit canal principal 105. La dérivation de contournement 115 comporte un puits 116 qui créé une perte de charge à ce niveau ce qui entraîne le liquide 4 à choisir le cheminement le plus aisé, à savoir le canal secondaire 108.

Le volume total de liquide 4 transféré au sein de la carte 101 est supérieur au volume total de toutes les cavités 109, 110 et 111, et est inférieur ou égal à la somme du volume total de toutes lesdites cavités 109, 110 et 111, de tous les canaux et dérivations et du volume du trop-plein 104. Dans un mode de réalisation, le niveau du liquide 4 présent dans ledit trop-plein 104, et qui s'écoule à ce niveau par gravité, est inférieur au point d'insertion de la sortie 127 sur le trop-plein 104. Le but est de permettre à l'air, une fois que le liquide 4 a rempli l'ensemble des cavités 109, 110 et 111, de remplacer ledit liquide 4 dans le canal principal d'amenée 105, afin de créer une isolation entre toutes lesdites cavités 109, 110 et 111.

Préférentiellement, le volume total de liquide 4 transféré au sein de la carte 101 est inférieur ou égal à la somme du volume total de toutes lesdites cavités 109, 110 et 111. De ce fait, il est possible de purger par de l'air, ou tout autre fluide inerte pour les analyses que l'on doit effectuer, le canal principal 105 de tout liquide 4 une fois que toutes les cavités 109, 110 et 111 ont été remplies. Cette purge permet d'isoler le contenu de toutes les cavités les unes des autres.

Comme cela a été précisé plus haut, les cavités de grande taille 109 et les cavités de taille moyenne 110 fonctionnent par écoulement du liquide 4 par gravité, alors que les cavités de petite taille 111 fonctionnent par écoulement par capillarité. D'ailleurs, les ordres de grandeur, qui seront donnés par la suite quant aux dimensions internes et externes de la carte 101, mettent en évidence ces phénomènes.

Sur les figures 14, 15 et 16, on note que pour faciliter encore le transfert vers les cavités de grande taille 109 et de taille moyenne 110, celles-ci 109 et 110 sont équipées d'un moyen de drainage 119 de forme tout à fait particulière en fonction de la distance le séparant de l'entrée de ladite cavité 109 ou 110, correspondant au canal secondaire 106 ou 107. Ainsi, alors qu'il 119 a une forme d'épaulement au plus près de cette entrée, avec une arête entre ce moyen 119 et la paroi latérale de la cavité 109 ou 110, cet angle disparaît progressivement pour faire place à une courbe qui va jusqu'à rejoindre le fond 120 du corps 102 de ladite carte 101.

Les dimensions de cette carte 101 sont données à titre d'exemple indicatif et non limitatif. Ainsi la carte 101 a une largeur de 108 mm, une longueur de 118 mm, et une épaisseur de 4 mm. Les autres dimensions sont les suivantes :
- le canal principal 105, et donc les dérivations 115, a une section en U dont l'ouverture est fermée par le film 35, la largeur et la profondeur de ce U sont de 0,5 mm,
- les dérivations supérieures 113 et les dérivations inférieures 117 ont une section en U dont l'ouverture est fermée par le film 35, la largeur et la profondeur de ce U sont de 0,2 mm,
- la profondeur, la longueur et la largeur des cavités de grande taille 109 sont respectivement de 3,1 mm, de 20 mm et de 5,5 mm,
- la profondeur, la longueur et la largeur des cavités de taille moyenne 110 sont respectivement de 1,8 mm, de 6 mm et de 3,5 mm,
- la profondeur, la longueur et la largeur des cavités de petite taille 111 sont respectivement de 1 mm, de 3 mm et de 1 mm,
- la profondeur, la longueur et la largeur du trop-plein 104 sont respectivement de 3,6 mm, de 25 mm et de 24 mm,
- le diamètre des puits 112, au niveau des cavités 109, est de 2 mm, il s'agit d'une calotte sphérique d'une profondeur de 1 mm, réalisée par une fraise en forme de boule de 2 mm de diamètre,
- le diamètre des puits 112, au niveau des cavités 110, est de 1,5 mm, de même pour le diamètre des puits 114, au niveau des cavités 109 et 110, et des puits 116 et 118 des cavités 111, il s'agit d'une calotte sphérique d'une profondeur de 0,75 mm, réalisée par une fraise en forme de boule de 1,5 mm de diamètre,
- le canal de sortie 127 à une section en U dont l'ouverture est fermée par le film 35, la largeur et la profondeur de ce U sont respectivement de 1 mm et de 0,5 mm, et
- les puits ralentisseurs 122, 123, 124, 125 et 126 ont tous un diamètre de 3 mm, il s'agit d'une calotte sphérique d'une profondeur de 1,5 mm, réalisée par une fraise en forme de boule de 3 mm de diamètre.

La procédure permettant de dénombrer les micro-organismes contenus dans un échantillon biologique 4 peut être utilisée dans le domaine alimentaire. Dans l'exemple présenté ci-après, la procédure est la suivante.

Elle consiste tout d'abord à préparer un échantillon 4 liquide. Ainsi, pour un poids de sensiblement 10 grammes d'un produit alimentaire, on ajoute dans un sac Stomacher avec filtre 90 ml d'eau peptonée. On homogénéise l'échantillon dans le sac Stomacher pendant 1 à 2 minutes. On obtient ainsi une première dilution 10⁻¹. On effectue des dilutions supplémentaires en transférant 1ml du liquide contenu dans ledit sac Stomacher dans un flacon contenant 49 ml d'un milieu de culture, puis on homogénéise. La dilution est alors de 2. 10⁻³, soit au 1/500.

Sachant que le volume des cavités 9 de grande de taille est de 200 µl, des cavités 10 de moyenne taille est de 20 µl, et des cavités 11 de petite taille est de 2 µl, étant donné qu'il y a vingt cavités 9, 10 ou 11 de chaque taille, le volume total desdites cavités 9, 10 ou 11 est de 4440 µl. Il convient de noter que ces chiffres sont donnés à titre d'exemple. Il est par exemple tout à fait possible d'avoir un nombre de cavités 9, 10 et 11 qui est variable d'une taille de cavités à l'autre.

Pour respecter le rapport entre le volume de liquide biologique 4 et le volume total de toutes les cavités, de un pour deux précédemment établi. on transfère donc 2 220 µl de ce flacon dans un tube 32. Un tuyau souple 33 est soit présent au niveau de la carte 1 et forme avec celle-ci le consommable, soit relié à ladite carte avant son utilisation. On place le tube 32 verticalement sur un support et on relie, par le tuyau 33 souple, le contenu du tube 32 à la zone d'injection 3 de la carte 1. Puis on place l'ensemble, ainsi constitué et représenté à la figure 5, sous vide, afin que l'échantillon 4 contenu dans ledit tube 32 soit prêt à être transféré en totalité à l'intérieur des cavités d'analyse 9, 10 ou 11. Ensuite, on casse la dépression progressivement, c'est-à-dire en au moins 10 secondes.

Ainsi, la figure 9 montre bien que la dépression exercée pour obtenir un vide adéquat, même partiel, doit être de sensiblement de 850 millibars et est atteinte en une minute. De même, le retour à la pression normale s'effectue progressivement en une minute. Ensuite, on retire l'ensemble du dispositif de la chambre réalisant le vide. On ferme hermétiquement la carte d'analyse 1 en coupant et en scellant au niveau du tuyau 33, à l'endroit où celui-ci affleure la surface latérale de ladite carte 1, et on retire ensuite le tube 32. Puis, on laisse la carte en position verticale. Ce dernier point n'est pas d'une absolue nécessité. Dans cette position, on incube les différentes cartes 1 pendant environ 24 heures à 30 °C.

On a ainsi réparti dans chacune des cavités 9, 10 et 11 respectivement 100, 10 et 1 µl de l'échantillon biologique.

Enfin, il est possible de lire la carte 1 en comptabilisant le nombre de cavité(s) d'analyse 9, 10 et/ou 11 qui ont été modifiées au niveau de leur densité optique ou de leur coloration, dans le cas de l'utilisation d'un marqueur fluorescent ou chromogène. Dans tous les cas de figure, cela correspond à un développement du type de micro-organismes que l'on voulait révéler. En fonction du nombre de cavités d'analyse 9 de grande taille, de cavités 10 de taille moyenne et de cavités 11 de petite taille qui ont changé d'aspect, il est possible de se reporter au tableau 1, qui suit, pour déterminer la quantité exacte du micro-organisme à l'intérieur de l'échantillon de départ.

On calcule le nombre le plus probable (NPP) de bactéries dans un échantillon par une méthode statistique, expliquée par R. J. Parnow (1972); Cf. PARNOW RJ, 1972 - Computer program estimates bacterial densities by means of the most probable numbers, Food Technology 7, 56-62. Cependant, cette méthode donne la totalité des combinaisons existantes pour les trois nombres de tubes positifs, or il y a des combinaisons qui sont hautement improbables ou statistiquement incorrectes. Par exemple, il est inconcevable de n'avoir aucun positif dans les cavités contenant 100 µl d'échantillon (première dilution) et d'en avoir vingt dans les cavités contenant 1 µl d'échantillon (troisième dilution).

Ainsi on associe la probabilité d'apparition à chaque combinaison de positif(s). On utilise pour cela la méthode de J. C. De Man (1975); Cf. DE MAN J, 1975 - The probability of most probable numbers, European Journal of Applied Microbiology, 1, 67-68; puis toujours selon cet auteur, on définit différentes combinaisons de positifs :
- la catégorie 1 rassemble toutes les combinaisons de la plus haute probabilité, dont la somme des probabilités est de 95%,
- la catégorie 2 rassemble toutes les combinaisons dont la somme des probabilités représente les 4% suivants, et
- la catégorie 3 rassemble toutes les combinaisons dont la somme des probabilités représente le 1% restant.

Dans le tableau 1 suivant, les combinaisons de la catégorie 1 sont seules à figurer, avec les NPP qui y sont associées. Le tableau ne tenant compte d'aucune dilution, il faut donc multiplier le NPP trouvé dans ce tableau 1 par le facteur total de dilution pour obtenir le niveau de contamination de l'échantillon initial.

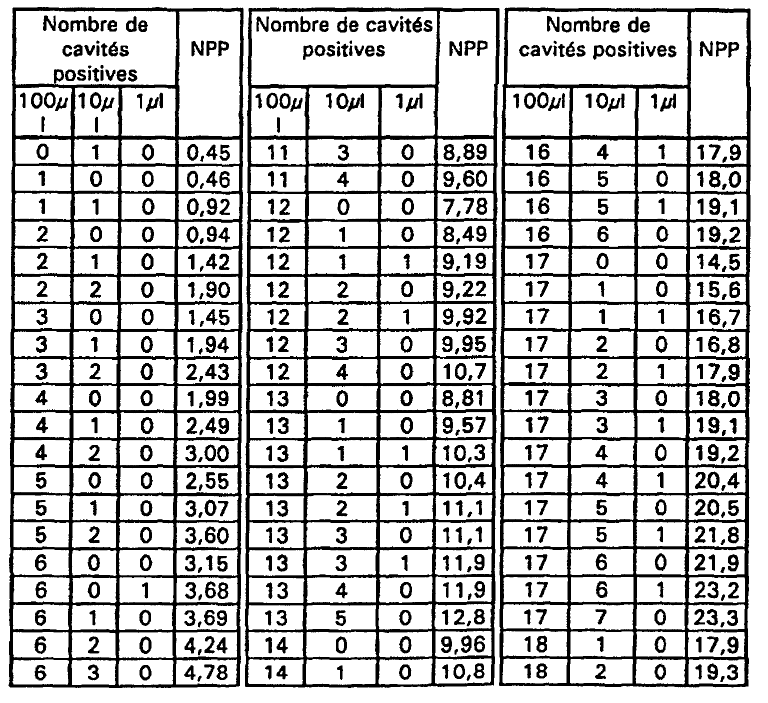

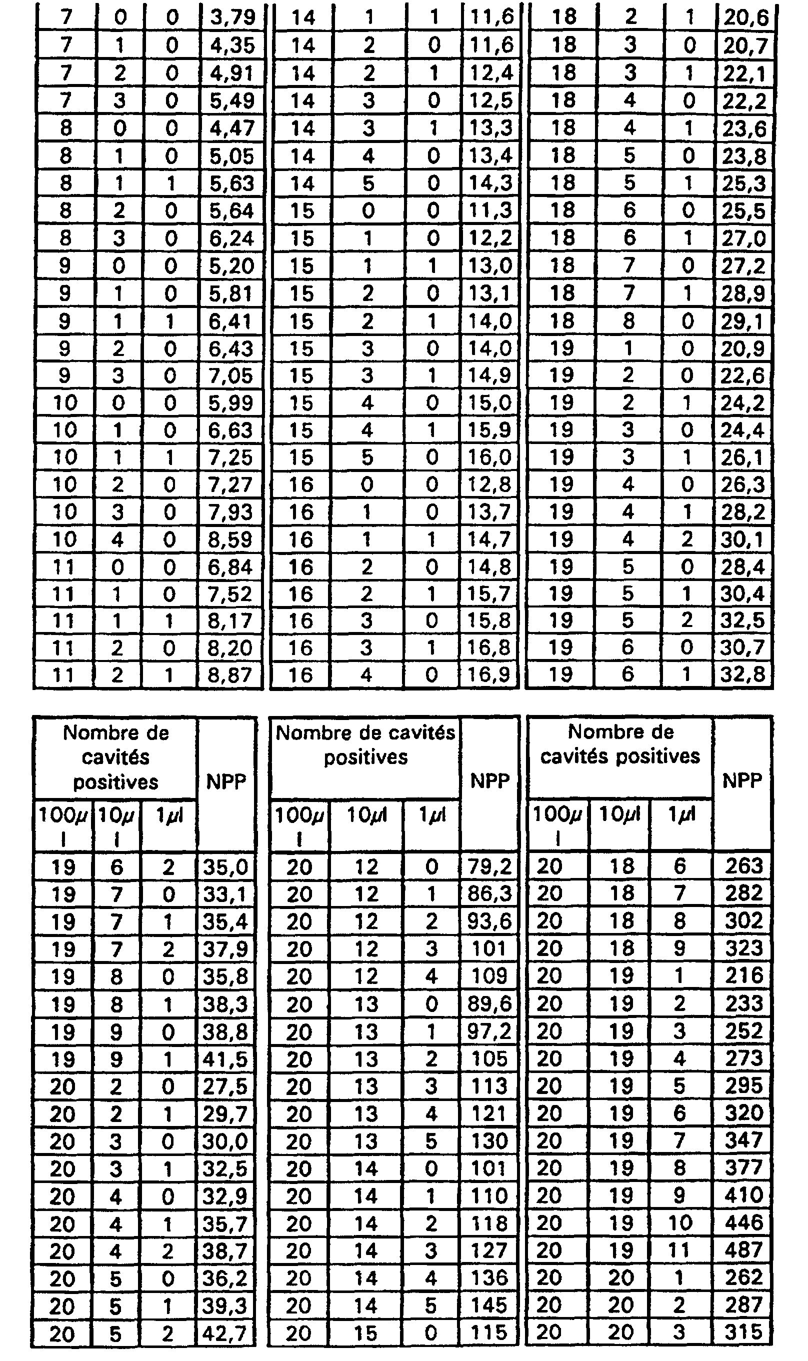

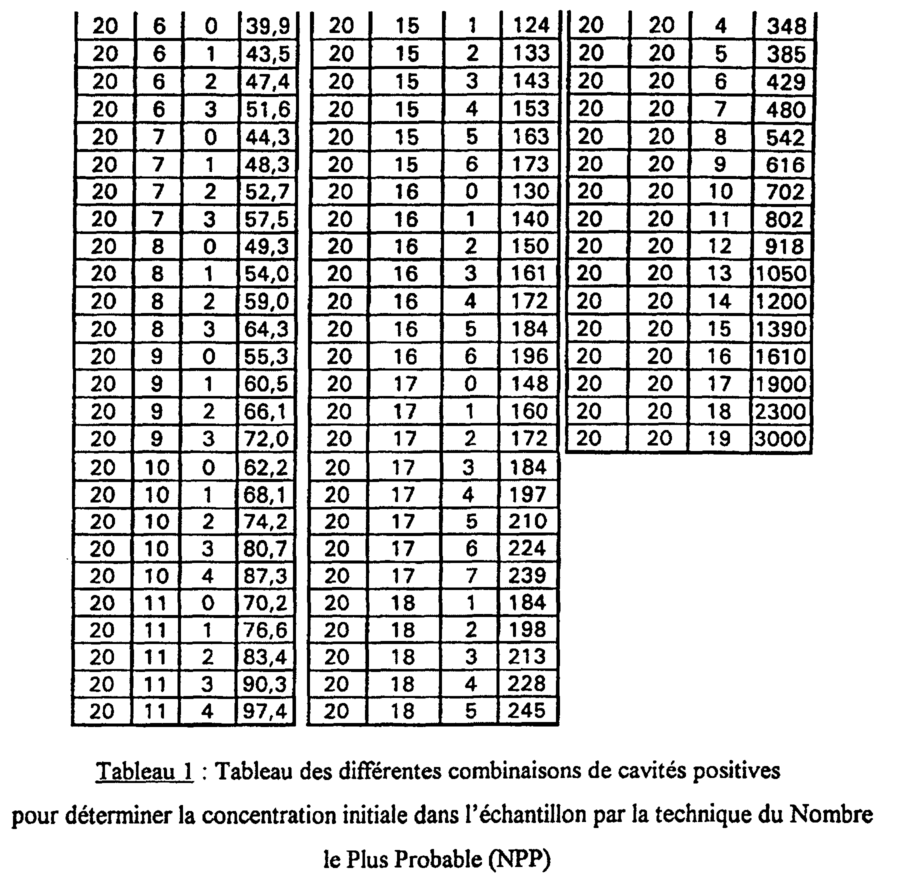

Il est tout à fait possible que la distribution des micro-organismes, surtout s'ils sont peu nombreux, ne se fasse pas aussi régulièrement que dans les exemples ci-dessous.

Si l'on se réfère à la figure 3, on remarque que l'ensemble des cavités d'analyse 9 présente une croissance microbienne. Par contre, au niveau des cavités d'analyse 11 de petite taille, aucune croissance n'est repérée. Dans le cas des cavités 10 de taille moyenne, seulement onze des cavités, sur les vingt, présentent une croissance. Si l'on se reporte alors au tableau 1, la combinaison 20/11/0 donne une Unité Formant Colonie par gramme de produit (UFC/g) de 70 UFC/g. Connaissant la dilution de départ, qui est de 1/500, il est possible de déduire que la concentration initiale en micro-organismes était de 70 x 500, c'est-à-dire 35000 UFC/g.

Selon la figure 4, dix-huit des cavités d'analyse 9 présentent une croissance microbienne. De même, trois des cavités d'analyse 10 et une des cavités d'analyse 11 présentent une croissance. On en déduit, de la même façon que pour l'exemple précédent, que la concentration de départ était de 22 x 500, soit 11000 UFC/g.

Selon la figure 10, six des vingt cavités d'analyse 9 présentent une croissance microbienne. A l'opposé, aucune des cavités 11 n'est modifiée, alors que seules deux des cavités d'analyse 10 présentent une croissance. On en déduit donc que pour cet échantillon dilué 500 fois, la concentration initiale était de 2100 UFC/g.

A la lecture du tableau, on comprend que pour un échantillon dilué 500 fois, il sera possible de déterminer une concentration initiale en micro-organismes comprise entre 225 et 1,5 x 10⁶ UFC/g, avec une seule carte d'analyse 1 ou 21, alors que l'état de la technique utilise généralement quatre consommables pour obtenir une telle amplitude dans les résultats. Il est également possible de déterminer une concentration qui serait inférieure ou supérieure à ces valeurs sans toutefois connaître la concentration exacte. Il est alors possible de faire une nouvelle dilution adaptée au précédent résultat afin d'avoir un résultat final précis.

Il est bien entendu possible d'avoir une carte d'analyse différente, qui comporte quatre, cinq tailles différentes de cavités, voire plus, et ce afin d'augmenter le champ d'investigation de la carte.

Bien entendu, il est possible de faire de la numération d'autres entités ou matériels biologiques telles que les acides nucléiques (ADN ou ARN), les anticorps, les antigènes. La révélation peut être réalisée par des anticorps anti-entités biologiques, qui sont marqués.

Il est possible d'ajouter dans le tube 32 un liquide, non miscible, de densité plus faible que l'échantillon 4, qui sera aspiré après ledit échantillon 4 et qui servira d'isolant entre les différentes quantités réparties dans chaque cavité. Il est également possible d'avoir deux tubes 32, l'un contenant l'échantillon biologique, l'autre contenant le fluide isolant, et de plonger le tuyau 33 alternativement dans le premier tube 32 puis dans le second tube 33. Il est enfin possible d'avoir sur la carte 1 deux tuyaux 33. Le volume total des cavités 9, 10 et 11 étant de 4440 µl, le volume de l'échantillon 4 étant de 2220 µl, le volume du liquide isolant pourra être de 2220 µl. Il pourra être inférieur si l'on désire qu'il y ait de l'air dans la carte 1 une fois scellée, ou supérieur si l'on veut être sûr du remplissage complet de la carte par l'échantillon 4 et le liquide isolant. Dans ce dernier cas, cette technique peut être intéressante pour la croissance de micro-organismes anaérobies.

Afin de déterminer exactement le volume de l'échantillon 4 qui doit être transféré dans la carte 1, trois possibilités existent qui ne font pas appel à une automatisation.

La première possibilité concerne le remplissage du tube 32 par la quantité exacte de l'échantillon 4. Le tuyau souple 33 ayant son extrémité libre 34 au niveau le plus inférieur dudit échantillon 4, la totalité de l'échantillon 4 est transférée.

La deuxième possibilité concerne le remplissage de la carte 1 par une quantité d'échantillon 4 déterminée par la position de l'extrémité libre 34 immergée dans ledit échantillon 4, au sein dudit tube 32. L'échantillon 4 aspiré correspond à la colonne de liquide 4 située au-dessus de ladite extrémité 34, et seule cette colonne de liquide supérieure est transférée.

Enfin, la troisième possibilité concerne le remplissage du tube 32 par une quantité très importante d'échantillon. Le transfert en cassant la dépression est réalisé jusqu'à ce que le manipulateur retire directement ou indirectement l'extrémité 34 du tuyau souple 33 de l'échantillon 4, tout en permettant la continuation du transfert des fractions dudit échantillon 4 vers les cavités 9, 10 et 11.

Il est donc aisé de comprendre que, lorsque le transfert de l'échantillon 4 s'effectue, le prélèvement a lieu, dans un premier temps, au niveau dudit échantillon 4. C'est donc le niveau de l'échantillon 4 qui diminue jusqu'à ce que l'extrémité libre 34 du tuyau souple 33 se retrouve dans le fluide isolant. C'est alors, dans un second temps, ce fluide qui est transféré vers la carte 1. Du fait de sa densité plus faible que celle de l'échantillon 4 et de la position de ladite carte 1, comme représentée à la figure 5, ledit fluide va faire office de barrière physique et biologique pour les différentes petites quantités d'échantillon 4 distribuées dans les cavités 9, 10 et 11.

Il est également tout à fait possible d'utiliser un agent gélifiant dans le milieu. Cette technique peut faciliter l'isolation entre les différentes cavités 9, 10 et 11 (Fig. 1) ou 29, 30 et 31 (Fig. 2) par un fluide gazeux, tel que de l'air.

Pour contrôler que le volume distribué dans chaque cavité 9, 10 ou 11 est sensiblement identique entre cavités de mêmes tailles et entre cavités de tailles différentes, des tests ont été réalisés.

Ainsi avec les cavités 11 de petite taille, trois essais ont été réalisés pour les vingt puits. Les tests ont été réalisés à partir de 2 220 µl d'eau déminéralisée avec une dépression de 875 millibars. Les résultats sont représentés sur le tableau 2 et sont les suivants :

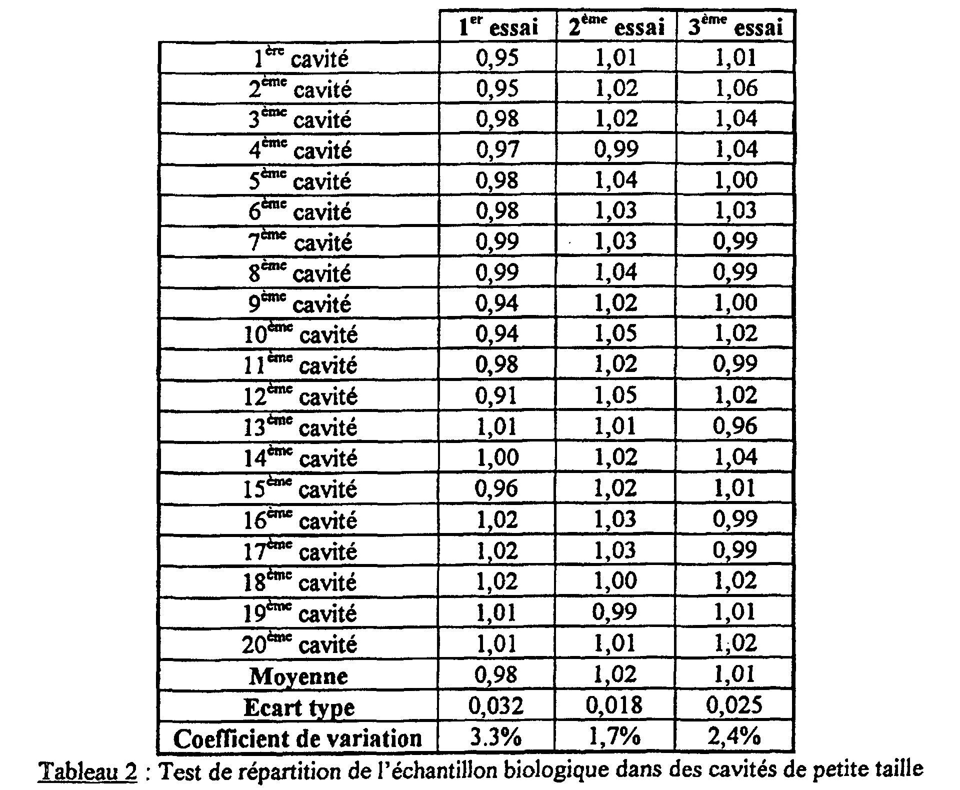

De même avec les cavités 10 de taille moyenne, trois essais ont été réalisés pour les vingt puits. Les tests ont été réalisés à partir de 2220 µl d'eau déminéralisée avec une dépression de 875 millibars. Les résultats sont représentées sur le tableau 3 et sont les suivants :

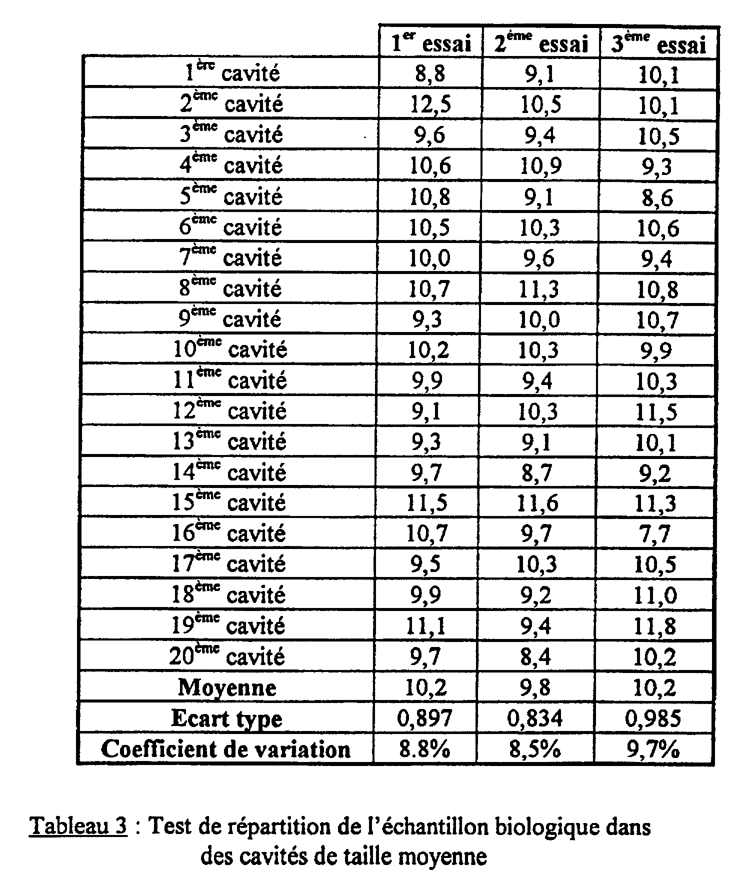

De manière identique aux deux cas précédents, trois essais, avec les cavités 9 de grande taille, ont été réalisés pour les vingt puits. Les tests ont été réalisés dans les mêmes conditions que précédemment, c'est-à-dire à partir de 2220 µl d'eau déminéralisée avec une dépression de 875 millibars. Les résultats sont présentés dans le tableau 4 qui suit :

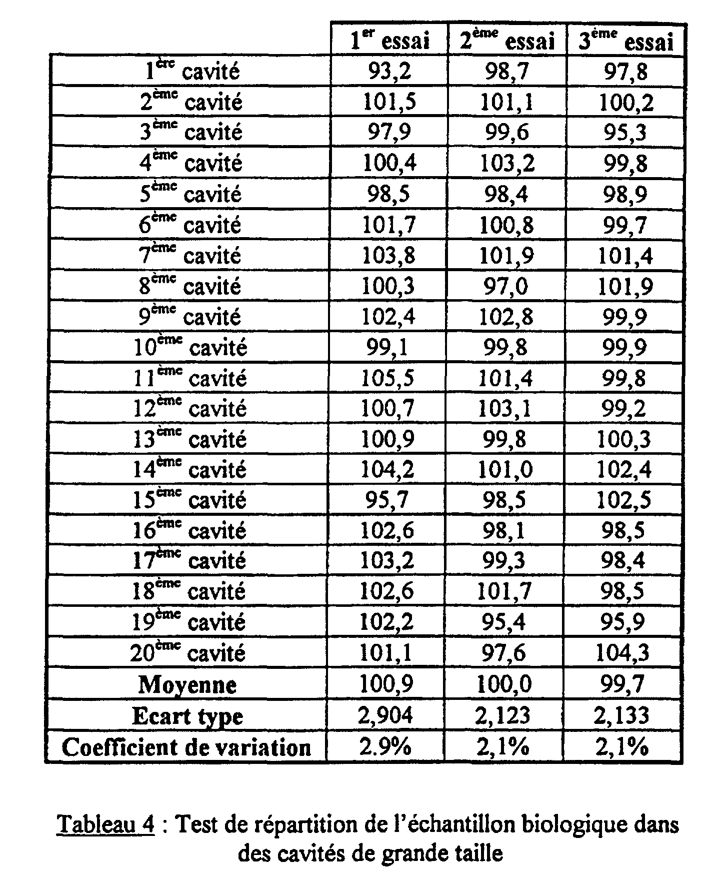

On remarque d'une part que le volume présent dans chaque puits ou cavité 9, 10 ou 11 de même taille est relativement constant, puisque la moyenne entre les trois essais est sensiblement constante, que l'écart type et le coefficient de variation sont faibles.

En fait, l'écart type est constant quel que soit le volume ; de ce fait il y a une diminution du coefficient de variation, qui est proportionnelle à l'augmentation du volume de liquide contenu dans l'échantillon. En fait ces résultats sont dus aux problèmes de précision des mesures de liquides présents dans chaque puits ou cavité 9, 10 ou 11, après la distribution de l'échantillon de départ.

Quoiqu'il en soit ces résultats sont particulièrement intéressants, et permettent l'utilisation de la méthode de calcul de la concentration de tout matériel biologique, tel qu'acides nucléiques, anticorps ou micro-organismes, présent dans ledit échantillon biologique 4 de départ.

La carte d'analyse 1 peut également être utilisée dans le domaine médical pour effectuer des numérations de micro-organismes dans les liquides biologiques.

L'exemple suivant décrit le protocole pouvant être employé dans le cadre d'une analyse d'urine pour effectuer la numération des germes urinaires. On réalise une dilution de l'urine prélevée en transférant (après homogénéisation) du récipient de prélèvement, une dose calibrée de 10µl dans un tube contenant 5ml d'un milieu de culture.

Après l'homogénéisation, on obtient une dilution à 2. 10⁻³ soit au 1/500. On transfère ensuite 2220 µl de cette dilution dans le tube 32. La carte 1 est ensuite remplie puis incubée et enfin lue de la même façon que dans les exemples décrits précédemment. La température d'incubation sera cependant plus élevée et proche de 37°C.

En utilisant ce protocole, on peut donc dénombrer les germes urinaires lorsque la contamination de l'urine est comprise entre 225 UFC/ml et 1,5. 10⁶ UFC/ml.

### REFERENCES

- 1.: Carte d'analyse
- 2.: Corps de la carte d'analyse 1
- 3.: Zone d'injection dans la carte 1
- 4.: Liquide ou échantillon biologique à analyser
- 5.: Canal principal d'amenée
- 6.: Canaux secondaires
- 7.: Puits correspondant à chaque canal 6 ou moyen de distribution
- 8.: Canaux terminaux
- 9.: Cavités d'analyse terminales, de grande taille, correspondant à un canal 8
- 10.: Cavités d'analyse terminales, de taille moyenne, correspondant à un canal 8
- 11.: Cavités d'analyse terminales, de petite taille, correspondant à un canal 8
- 12.: Puits ou moyen de distribution principal
- 13.: Zone d'injection latérale
- 14.: Rainure circulaire dans le corps 2
- 15.: Epaulement du tuyau 33 coopérant avec la rainure 14
- 21.: Carte d'analyse
- 22.: Corps de la carte d'analyse 21
- 23.: Zone d'injection dans la carte 21
- 25.: Canaux d'amenée
- 27.: Puits correspondant à chaque canal 25 ou moyen de distribution
- 28.: Canaux terminaux
- 29.: Cavités d'analyse terminales, de grande taille, correspondant à un canal 28
- 30.: Cavités d'analyse terminales, de taille moyenne, correspondant à un canal 28
- 31.: Cavités d'analyse terminales, de petite taille, correspondant à un canal 28
- 32.: Tube contenant l'échantillon 4
- 33.: Tuyau reliant la zone 3 au tube 32
- 34.: Extrémité libre du tuyau 33
- 35.: Film transparent adhésif
- 101.: Carte d'analyse
- 102.: Corps de la carte d'analyse 101
- 103.: Zone d'injection dans la carte 101
- 104.: Trop-plein
- 105.: Canal principal d'amenée
- 106.: Canaux secondaires des cavités 109
- 107.: Canaux secondaires des cavités 110
- 108.: Canaux secondaires des cavités 111
- 109.: Cavités d'analyse terminales, de grande taille, correspondant à un canal 106
- 110.: Cavités d'analyse terminales, de taille moyenne, correspondant à un canal 107
- 111.: Cavités d'analyse terminales, de petite taille, correspondant à un canal 108
- 112.: Puits pour retarder le passage de l'échantillon 4 dans le canal principal 105 au niveau des cavités 109 ou 110
- 113.: Dérivation supérieure des cavités 109 ou 110
- 114.: Puits pour empêcher le passage de l'échantillon 4 dans les dérivations 113
- 115.: Dérivation de contournement des cavités 109, 110 ou 111
- 116.: Puits pour retarder le passage de l'échantillon 4 dans le canal principal 105 au niveau des cavités 111
- 117.: Dérivation inférieure des cavités 111
- 118.: Puits pour empêcher le passage de l'échantillon 4 dans la dérivation 117
- 119.: Moyen de drainage de l'échantillon 4
- 120.: Fond du corps 102 de la carte 101 au niveau d'une cavité 109, 110 ou 111
- 121.: Vanne entre la zone d'injection 103 et le canal principal 105
- 122.: Vanne entre les deux niveaux des cavités 109
- 123.: Vanne entre les cavités 109 et les cavités 110
- 124.: Vanne entre les deux niveaux des cavités 110
- 125.: Vanne entre les cavités 110 et les cavités 111
- 126.: Vanne entre les deux niveaux des cavités 111
- 127.: Sortie du trop-plein 104
- F1.: Arrivée de l'échantillon 4 par le canal principal 5 dans un canal secondaire 106, 107 ou 108
- F2.: Arrivée de l'échantillon 4 par un canal secondaire 106, 107 ou 108 dans une cavité 109, 110 ou 111
- F3.: Transfert d'air par la dérivation supérieure 113 des cavités 109 ou 110
- F4.: Transfert de l'échantillon 4 par le canal principal 5 au niveau des cavités 109, 110 ou 111
- F5.: Sortie de l'échantillon 4 par le canal principal 5 au niveau des cavités 109, 110 ou 111
- F6.: Transfert d'air et/ou d'échantillon 4 par la dérivation inférieure 117 des cavités 111

## Revendications

1. Carte d'analyse (1) qui comprend un corps (2) constitué par :
- une zone d'injection (3) dans la carte (1) d'un liquide biologique (4) à analyser,
- un canal principal d'amenée (5) du liquide biologique (4) provenant de la zone d'injection (3), et
- au moins deux canaux secondaires (6) situés dans le prolongement du canal principal (5),
**caractérisée par** le fait qu'elle comprend également :
- au moins un puits (7) correspondant à chaque canal secondaire (6), chaque puits (7) constitue, d'une part, un moyen de distribution d'un volume déterminé dudit liquide biologique (4), dans au moins deux cavités d'analyse terminales (9, 10 ou 11), via un canal terminal (8) pour chaque cavité d'analyse (9, 10 ou 11), et d'autre part, un moyen de stockage d'un volume d'un fluide inerte, qui isole les cavités d'analyse (9, 10 et 11) entre elles, et
- une dépression cassable au sein de la carte (1), c'est-à-dire dans le volume intérieur de cette carte (1) constitué par les canaux (5, 6 et 8), les puits (7) et les cavités (9, 10 et 11), soit en plus au voisinage de ladite carte (1), c'est-à-dire le volume adjacent autour de la carte (1).

2. Carte d'analyse (21) qui comprend un corps (22) constitué par :
- une zone d'injection (23) dans la carte (21) d'un liquide biologique à analyser, et
- au moins deux canaux d'amenée (25) du liquide biologique provenant de la zone d'injection (23),
**caractérisée par le fait qu'**elle comprend également :
- au moins un puits (27) correspondant à chaque canal d'amenée (25), chaque puits (27) constitue, d'une part, un moyen de distribution, d'un volume déterminé dudit liquide biologique, dans au moins deux cavités d'analyse terminales (29, 30 ou 31), via un canal terminal (28) pour chaque cavité d'analyse (29, 30 ou 31), et d'autre part, un moyen de stockage d'un volume d'un fluide inerte, qui isole les cavités d'analyse (29, 30 et 31) entre elles, et
- une dépression cassable au sein de la carte (21), c'est-à-dire dans le volume intérieur de cette carte (21) constitué par les canaux (25 et 28), les puits (27) et les cavités (29, 30 et 31), soit en plus au voisinage de ladite carte (21), c'est-à-dire le volume adjacent autour de la carte (21).

3. Carte, selon l'une quelconque des revendications 1 ou 2, **caractérisée par le fait que** la distribution du liquide biologique (4) dans les cavités d'analyse terminales (9, 10 et 11 ou 29, 30 ou 31) s'effectue en parallèle.

4. Carte d'analyse (101) qui comprend un corps (102) constitué par :
- une zone d'injection (103) dans la carte (101) d'un liquide biologique (4) à analyser,
- un canal principal d'amenée (105) du liquide biologique (4) provenant de la zone d'injection (103), et
- au moins deux canaux secondaires (106, 107 et/ou 108) situés dans le prolongement du canal principal (105), et
**caractérisée par le fait que** la carte (101) comprend également :
- au moins une cavité (109, 110 et/ou 111) correspondant à chaque canal secondaire (106, 107 ou 108), chaque cavité (109, 110 ou 111) constitue un moyen de réception d'un volume déterminé dudit liquide biologique (4), et
- une chambre pour recevoir un volume de trop-plein correspondant au liquide (4) présent au niveau du canal (105), qui isole les cavités d'analyse (109, 110 et 111) entre elles.

5. Carte, selon la revendication 4, **caractérisée par le fait que** la distribution du liquide biologique (4) dans les cavités d'analyse (109, 110 ou 111) s'effectue en série et est suivie d'une purge.

6. Carte, selon la revendication 1, **caractérisée par le fait qu'**un puits (12), faisant office de moyen de distribution principal du liquide biologique (4), est présent entre le canal principal (5), d'une part, et les canaux secondaires (6), d'autre part.

7. Carte, selon l'une quelconque des revendications 1, 2 ou 6, **caractérisée par le fait que** les volumes des canaux secondaires (6) ou des canaux d'amenée (25) sont tous identiques.

8. Carte, selon l'une quelconque des revendications 1 à 7, **caractérisée par le fait que** les cavités d'analyse terminales (9, 10 et/ou 11 ou bien 29, 30 et/ou 31 ou bien 109, 110 et/ou 111) sont d'au moins deux volumes différents.

9. Carte, selon l'une quelconque des revendications 1 à 8, **caractérisée par le fait que** les cavités d'analyse terminales (9, 10 et/ou 11 ou bien 29, 30 et/ou 31 ou bien 109, 110 et/ou 111) sont de trois volumes différents.

10. Carte, selon l'une quelconque des revendications 8 ou 9, **caractérisée par le fait que** le rapport existant entre deux cavités de volumes différents (9 et 10 ou 10 et 11 ou 9 et 11 ou bien 29 et 30 ou 30 et 31 ou 29 et 31 ou bien 109 et 110 ou 110 et 111 ou 109 et 111) est compris entre 1/5 et 1/20, 1/50 et 1/200 ou 1/500 et 1/2000, et notamment 1/10, 1/100 bu 1/1000.

11. Carte, selon l'une quelconque des revendications 1 à 9, **caractérisée par le fait que** les cavités (9, 10 et/ou 11 ou bien 29, 30 et/ou 31 ou bien 109, 110 et/ou 111) d'un même volume sont disposées géométriquement, soit de façon alignées, soit de façon concentrique, pour faciliter la lecture.

12. Procédé de remplissage d'une carte d'analyse (1 ou 21), telle qu'exposée selon l'une quelconque des revendications 1 à 3, 6 ou 7, **caractérisé en ce qu'**il consiste à :
- relier la zone d'injection (3 ou 23) de la carte (1 ou 21) à un volume d'un liquide biologique (4) à analyser,
- créer une dépression au sein et/ou au voisinage de ladite carte (I ou 21),
- casser la dépression afin, d'une part, de transférer le liquide biologique (4) dans les cavités d'analyse (9, 10 et/ou 11 ou bien 29, 30 et/ou 31), et, d'autre part, d'isoler par un fluide inerte les différentes fractions de l'échantillon, issues dudit liquide biologique (4), qui sont présentes dans chacune desdites cavités d'analyse (9, 10 ou 11 ou bien 29, 30 ou 31), et
- analyser le résultat obtenu afin de déterminer la quantité de micro-organismes ou de tout autre élément biologique, tel qu'acides nucléiques, anticorps, présent dans l'échantillon biologique (4) de départ.

13. Procédé de remplissage d'une carte d'analyse (101), telle qu'exposée selon l'une quelconque des revendications 4 ou 5, **caractérisé en ce qu'**il consiste à :
- relier la zone d'injection (103) ou la sortie (127) de la carte (101) à un volume d'un liquide biologique (4) à analyser,
- créer une dépression ou une surpression au sein et/ou au voisinage de ladite carte (101), afin de transférer le liquide biologique (4) dans les cavités d'analyse (109, 110 et/ou 111),
- stopper la dépression ou la surpression,
- purger le canal principal (105) pour isoler par un fluide inerte les différentes fractions de l'échantillon, issues dudit liquide biologique (4), qui sont présentes dans chacune desdites cavités d'analyse (109, 110 ou 111), et
- analyser le résultat obtenu afin de déterminer la quantité de micro-organismes ou de tout autre élément biologique, tel qu'acides nucléiques, anticorps, présent dans l'échantillon biologique (4) de départ.

14. Procédé, selon la revendication 12, **caractérisé en ce que**, une fois que la dépression est cassée, le fluide isolant est en contact avec le liquide biologique (4) soit au niveau des canaux secondaires (6) ou des canaux d'amenée (25) soit au niveau des cavités d'analyse (9, 10 et/ou 11 ou bien 29, 30 et/ou 31).

15. Procédé, selon l'une quelconque des revendications 12 ou 14, **caractérisé en ce que** le volume du liquide biologique (4) à analyser est inférieur au volume total de toutes les cavités d'analyse (9, 10 et 11 ou bien 29, 30 et 31).

16. Procédé, selon la revendication 13, **caractérisé en ce que** le volume du liquide biologique (4) à analyser est supérieur ou égal au volume total de toutes les cavités d'analyse (109, 110 et 111), de tout le réseau fluidique (105 à 108 et 112 à 118) et du trop-plein (104).

17. Procédé, selon l'une quelconque des revendications 13 ou 16, **caractérisé en ce que** le volume du liquide biologique (4) à analyser est supérieur ou égal au volume total de toutes les cavités d'analyse (109, 110 et 111) et de tout le réseau fluidique (105 à 108 et 112 à 118).

18. Procédé, selon l'une quelconque des revendications 12, 14 et 15, **caractérisé en ce que** le rapport entre le volume du liquide biologique (4) à analyser et le volume total de toutes les cavités d'analyse (9, 10 et 11 ou bien 29, 30 et 31) est de 1/10 à 9/10, notamment de 4/10 à 6/10, et préférentiellement de 5/10.

19. Utilisation d'une carte d'analyse (1, 21 ou 101), selon l'une quelconque des revendications 1 à 11, pour la numération de micro-organismes suivant la technique du nombre le plus probable.

## Patentansprüche

1. Analysekarte (1), die einen Körper (2) aufweist, der aus folgendem gebildet ist, nämlich
- einer Zone zur Injektion (3) einer zu analysierenden biologischen Flüssigkeit (4) in die Karte (1),
- einem Hauptkanal zur Zuführung (5) der biologischen Flüssigkeit (4), die aus der Injektionszone (3) stammt, und
- zumindest zwei Ableitkanälen (6), die in der Verlängerung des Hauptkanals (5) angeordnet sind,
**dadurch gekennzeichnet, daß** die Karte gleichermaßen folgendes aufweist, nämlich
- zumindest eine, jedem Ableitkanal (6) entsprechende, Vertiefung (7), wobei jede Vertiefung (7) zum einen ein Mittel zur Verteilung eines bestimmten Volumens der biologischen Flüssigkeit (4) in zumindest zwei endseitige Analysekavitäten (9, 10 oder 11) über einen Endkanal (8) für jede Analysekavität (9, 10 oder 11) bildet, und zum anderen ein Speichermittel für ein Volumen eines inerten Fluids bildet, das die Analysekavitäten (9, 10 oder 11) untereinander isoliert, und
- einen aufhebbaren Unterdruck im Innern der Karte (1), d.h. in dem Innenvolumen dieser Karte (1), das durch die Kanäle (5, 6 und 8), die Vertiefungen (7) und die Kavitäten (9, 10 und 11) gebildet ist, der darüber hinaus in der Umgebung der Karte (1), d.h. dem angrenzenden Raum um die Karte (1) vorhanden sein kann.

2. Analysekarte (21), die einen Körper (22) aufweist, der aus folgendem gebildet ist, nämlich
- einer Zone zur Injektion (23) einer zu analysierenden biologischen Flüssigkeit in die Karte (21), und
- zumindest zwei Kanälen zur Zuführung (25) der biologischen Flüssigkeit, die aus der Injektionszone (23) stammt,
**dadurch gekennzeichnet, daß** die Karte gleichermaßen folgendes aufweist, nämlich
- zumindest eine, jedem Zuführungskanal (25) entsprechende, Vertiefung (27), wobei jede Vertiefung (27) zum einen ein Mittel zur Verteilung eines bestimmten Volumens der biologischen Flüssigkeit in zumindest zwei endseitige Analysekavitäten (29, 30 oder 31) über einen Endkanal (28) für jede Analysekavität (29, 30 oder 31) bildet, und zum anderen ein Speichermittel für ein Volumen eines inerten Fluids bildet, das die Analysekavitäten (29, 30 und 31) untereinander isoliert, und
- einen aufhebbaren Unterdruck im Innern der Karte (21), d.h. in dem Innenvolumen dieser Karte (21), das durch die Kanäle (25 und 28), die Vertiefungen (27) und die Kavitäten (29, 30 und 31) gebildet wird, der darüber hinaus in der Umgebung der Karte (21), d.h. dem angrenzenden Raum um die Karte (21) vorhanden sein kann.

3. Karte nach Anspruch 1 oder 2, **dadurch gekennzeichnet, daß** die Verteilung der biologischen Flüssigkeit (4) in die endseitigen Analysekavitäten (9, 10 und 11 oder 29, 30 oder 31) parallel bewirkt wird.

4. Analysekarte (101), die einen Körper (102) aufweist, der aus folgendem gebildet ist, nämlich
- einer Zone zur Injektion (103) einer zu analysierenden biologischen Flüssigkeit (4) in die Karte (101),
- einem Hauptkanal zur Zuführung (105) der biologischen Flüssigkeit (4), die aus der Injektionszone (103) stammt, und
- zumindest zwei Ableitkanälen (106, 107 und/oder 108), die in der Verlängerung des Hauptkanals (105) angeordnet sind,
**dadurch gekennzeichnet, daß** die Karte (101) gleichermaßen folgendes aufweist, nämlich
- zumindest eine, jedem Ableitkanal (106, 107 oder 108) entsprechende, Kavität (109, 110 und/oder 111), wobei jede Kavität (109, 110 oder 111) ein Aufnahmemittel für ein bestimmtes Volumen der biologischen Flüssigkeit (4) bildet, und
- eine Kammer zur Aufnahme eines Überschußvolumens, das der im Bereich des Kanals (105) vorhandenen Flüssigkeit (4) entspricht, und welche die Analysekavitäten (109, 110 und 111) untereinander isoliert.

5. Karte nach Anspruch 4, **dadurch gekennzeichnet, daß** die Verteilung der biologischen Flüssigkeit (4) in die Analysekavitäten (109, 110 oder 111) in Serie durchgeführt wird und darauf ein Abführen folgt.

6. Karte nach Anspruch 1, **dadurch gekennzeichnet, daß** eine Vertiefung (12), die als Hauptverteilmittel für die biologische Flüssigkeit (4) tätig ist, zwischen dem Hauptkanal (5) einerseits und den Ableitkanälen (6) andererseits vorhanden ist.

7. Karte nach einem der Ansprüche 1, 2 oder 6, **dadurch gekennzeichnet, daß** Volumina der Ableitkanäle (6) bzw. der Zuführungskanäle (25) sämtlich identisch sind.

8. Karte nach einem der Ansprüche 1 bis 7, **dadurch gekennzeichnet, daß** die endseitigen Analysekavitäten (9, 10 und/oder 11 oder auch 29, 30 und/oder 31 oder auch 109, 110 und/oder 111) zumindest zwei verschiedene Volumina haben.

9. Karte nach einem der Ansprüche 1 bis 8, **dadurch gekennzeichnet, daß** die endseitigen Analysekavitäten (9, 10 und/oder 11 oder auch 29, 30 und/oder 31 oder auch 109, 110 und/oder 111) drei verschiedene Volumina haben.

10. Karte nach Anspruch 8 oder 9, **dadurch gekennzeichnet, daß** das Verhältnis, das zwischen zwei Kavitäten von unterschiedlichen Volumina (9 und 10 oder 10 und 11 oder 9 und 11 oder auch 29 und 30 oder 30 und 31 oder 29 und 31 oder auch 109 und 110 oder 110 und 111 oder 109 und 111) vorherrscht, bei 1/5 bis 1/20, bei 1/50 bis 1/200 oder bei 1/500 bis 1/2000 liegt, und insbesondere 1/10, 1/100 oder 1/1000 beträgt.

11. Karte nach einem der Ansprüche 1 bis 9, **dadurch gekennzeichnet, daß** die Kavitäten (9, 10 und/oder 11 oder auch 29, 30 und/oder 31 oder auch 109, 110 und/oder 111) mit demselben Volumen geometrisch angeordnet sind, entweder aneinandergereiht, oder konzentrisch, um das Ablesen zu erleichtern.

12. Verfahren zum Befüllen einer Analysekarte (1 oder 21), nach einem der Ansprüche 1 bis 3, 6 oder 7 **dadurch gekennzeichnet, daß** es aus folgendem besteht, nämlich
- Verbinden der Injektionszone (3 oder 23) der Karte (1 oder 21) mit einem Volumen einer zu analysierenden biologischen Flüssigkeit (4),
- Errichten eines Unterdrucks innerhalb und/oder in der Umgebung der Karte (1 oder 21),
- Aufheben des Unterdrucks, um einerseits die biologische Flüssigkeit (4) in die Analysekavitäten (9, 10 und/oder 11 oder auch 29, 30 und/oder 31) zu überführen, und andererseits, um mittels eines inerten Fluids die verschiedenen Fraktionen der Probe, die aus der biologischen Flüssigkeit (4) stammen und die in jeder der Analysekavitäten (9, 10 oder 11 oder auch 29, 30 oder 31) vorhanden sind, zu isolieren, und
- Analysieren des erhaltenen Ergebnisses, um die Menge von Mikroorganismen oder jeglichen anderen in der biologischen Ausgangsprobe (4) vorhandenen biologischen Elements, wie z.B. Nukleinsäuren, Antikörper, zu bestimmen.

13. Verfahren zum Befüllen einer Analysekarte (101) nach Anspruch 4 oder 5, **dadurch gekennzeichnet, daß** es aus folgendem besteht, nämlich
- Verbinden der Injektionszone (103) oder des Ausgangs (127) der Karte (101) mit einem Volumen einer zu analysierenden biologischen Flüssigkeit (4),
- Errichten eines Unterdrucks oder eines Überdrucks innerhalb und/oder in der Umgebung der Karte (101), um die biologische Flüssigkeit (4) in die Analysekavitäten (109, 110 und/ oder 111) zu überführen,
- Abstellen des Unterdrucks oder des Überdrucks,
- Entleeren des Hauptkanals (105), um mittels eines inerten Fluids die verschiedenen Fraktionen der Probe, die aus der biologischen Flüssigkeit (4) stammen, und die in jeder der Analysekavitäten (109, 110 oder 111) vorhanden sind, zu isolieren, und
- Analysieren des erhaltenen Ergebnisses, um die Menge von Mikroorganismen oder jeglichen anderen in der biologischen Ausgangsprobe (4) vorhandenen biologischen Elements, wie z.B. Nukleinsäuren, Antikörper, zu bestimmen.

14. Verfahren nach Anspruch 12, **dadurch gekennzeichnet, daß**, wenn einmal der Unterdruck aufgehoben ist, das isolierende Fluid in Kontakt mit der biologischen Flüssigkeit (4) entweder im Bereich der Ableitkanäle (6) bzw. der Zuführungskanäle (25) oder im Bereich der Analysekavitäten (9, 10 und/oder 11 oder auch 29, 30 und/oder 31) ist.

15. Verfahren nach Anspruch 12 oder 14, **dadurch gekennzeichnet, daß** das Volumen der zu analysierenden Flüssigkeit (4) kleiner ist als das Gesamtvolumen von sämtlichen Analysekavitäten (9, 10 und 11 oder auch 29, 30 und 31).

16. Verfahren nach Anspruch 13, **dadurch gekennzeichnet, daß** das Volumen der zu analysierenden biologischen Flüssigkeit (4) größer oder gleich dem Gesamtvolumen von sämtlichen Analysekavitäten (109, 110 und 111), des gesamten Fluidiknetzes (105 bis 108 und 112 bis 118) und des Überlaufs (104) ist.

17. Verfahren nach Anspruch 13 oder 16, **dadurch gekennzeichnet, daß** das Volumen der zu analysierenden biologischen Flüssigkeit (4) größer oder gleich dem Gesamtvolumen von sämtlichen Analysekavitäten (109, 110 und 111) und des gesamten Fluidiknetzes (105 bis 108 und 112 bis 118) ist.

18. Verfahren nach einem der Ansprüche 12, 14 oder 15, **dadurch gekennzeichnet, daß** das Verhältnis zwischen dem Volumen der zu analysierenden biologischen Flüssigkeit (4) und dem Gesamtvolumen von sämtlichen Analysekavitäten (9, 10 und 11 oder auch 29, 30 und 31) bei 1/10 bis 9/10, insbesondere bei 4/10 bis 6/10, und vorzugsweise bei 5/10 liegt.

19. Verwendung einer Analysekarte (1, 21 oder 101) nach einem der Ansprüche 1 bis 11 zur Zählung von Mikroorganismen nach der Technik der wahrscheinlichsten Anzahl.

## Claims

1. Analysis card (1) which comprises a body (2) consisting of:
- a region (3) for injecting a biological liquid (4) to be analyzed into the card (1),
- a main feed channel (5) for the biological liquid (4) coming from the injection region (3), and
- at least two secondary channels (6) lying in the extension of the main channel (5),
**characterized in that** it also comprises:
- at least one well (7) corresponding to each secondary channel (6), each well (7) constitutes, on the one hand, a means of delivering a predetermined volume of said biological liquid (4) into at least two terminal analysis cavities (9, 10 or 11), via a terminal channel (8) for each analysis cavity (9, 10 or 11), and, on the other hand, a means of storing a volume of an inert fluid which isolates the analysis cavities (9, 10 and 11) from one another, and
- a vacuum that can be broken within the card (1), that is to say in the internal volume of this card (1) and consisting of the cavities (5, 6 and 8), the wells (7) and the cavities (9, 10 and 11), is also in the vicinity of said card (1), i.e. the adjacent volume around the card (1).

2. Analysis card (21) which comprises a body (22) consisting of:
- a region (23) for injecting a biological liquid to be analyzed into the card (21), and
- at least two feed channels (25) for the biological liquid coming from the injection region (23),
**characterized in that** it also comprises:
- at least one well (27) corresponding to each feed channel (25), each well (27) constitutes, on the one hand, a means of delivering a predetermined volume of said biological liquid into at least two terminal analysis cavities (29, 30 or 31), via a terminal channel (28) for each analysis cavity (29, 30 or 31), and, on the other hand, a means of storing a volume of an inert fluid which isolates the analysis cavities (29, 30 and 31) from one another, and
- a vacuum that can be broken within the card (21), i.e. in the internal volume of this card (21) formed by the channels (25 and 28), the wells (27) and the cavities (29, 30 and 31), is also in the vicinity of said card (21), i.e. the adjacent volume around the card (21).

3. Card according to either of claims 1 and 2, **characterized in that** the delivery of the biological liquid (4) into the terminal analysis cavities (9, 10 and 11 or 29, 30 or 31) takes place in parallel.

4. Analysis card (101) which comprises a body (102) consisting of:
- a region (103) for injecting a biological liquid (4) to be analyzed into the card (101),
- a main feed channel (105) for the biological liquid (4) coming from the injection region (103), and
- at least two secondary channels (106, 107 and/or 108) lying in the extension of the main channel (105), and
**characterized in that** the card (101) also comprises:
- at least one cavity (109, 110 and/or 111) corresponding to each secondary channel (106, 107 or 108), each cavity (109, 110 or 111) constitutes a means of receiving a predetermined volume of said biological liquid (4), and
- a chamber for receiving an overflow volume corresponding to the liquid (4) present in the channel (105), which isolates the analysis cavities (109, 110 and 111) from one another.

5. Card according to claim 4, **characterized in that** the delivery of the biological liquid (4) into the analysis cavities (109, 110 or 111) takes place in series and is followed by a purge.

6. Card according to claim 1, **characterized in that** a well (12), acting as a main means of delivering the biological liquid (4), is present between, on the one hand, the main channel (5) and, on the other hand, the secondary channels (6).

7. Card according to any one of claims 1, 2 or 6, **characterized in that** the volumes of the secondary channels (6) or of the feed channels (25) are all identical.

8. Card according to any one of claims 1 to 7 **characterized in that** the terminal analysis cavities (9, 10 and/or 11 or else 29, 30 and/or 31 or else 109, 110 and/or 111) are of at least two different volumes.

9. Card according to any one of claims 1 to 8, **characterized in that** the terminal analysis cavities (9, 10 and/or 11 or else 29, 30 and/or 31 or else 109, 110 and/or 111) are of three different volumes.

10. Card according to either of claims 8 and 9, **characterized in that** the ratio existing between two cavities of different volumes (9 and 10 or 10 and 11 or 9 and 11 or else 29 and 30 or 30 and 31 or 29 and 31 or else 109 and 110 or 110 and 111 or 109 and 111) is between 1/5 and 1/20, 1/50 and 1/200 or 1/500 and 1/2000, and especially 1/10, 1/100 or 1/1000.

11. Card according to any one of claims 1 to 9, **characterized in that** the cavities (9, 10 and/or 11 or else 29, 30 and/or 31 or else 109, 110 and/or 111) of the same volume are arranged geometrically, either in an aligned manner or a concentric manner, in order to facilitate the read-out.

12. Method of filling an analysis card (1 or 21), as presented according to any one of claims 1 to 3, 6 or 7, **characterized in that** it consists in:
- connecting the injection region (3 or 23) of the card (1 or 21) to a volume of a biological liquid (4) to be analyzed,
- creating a vacuum within and/or in the vicinity of said card (1 or 21),
- breaking the vacuum so as, on the one hand, to transfer the biological liquid (4) into the analysis cavities (9, 10 and/or 11 or else 29, 30 and/or 31) and, on the other hand, to isolate with an inert fluid the various fractions of the sample which come from said biological liquid (4) and which are present in each of said analysis cavities (9, 10 or 11 or else 29, 30 or 31) and
- analyzing the result obtained so as to determine the amount of microorganisms or of any other biological component, such as nucleic acids, antibodies, present in the initial biological sample (4).

13. Method of filling an analysis card (101) as presented according to either of claims 4 and 5,
**characterized in that** it consists in:
- connecting the injection region (103) or the outlet (127) of the card (101) to a volume of a biological liquid (4) to be analyzed,
- creating a vacuum or an overpressure within and/or in the vicinity of said card (101) so as to transfer the biological liquid (4) into the analysis cavities (109, 110 and/or 111),
- stopping the vacuum or the overpressure,
- purging the main channel (105) in order to isolate with an inert fluid the various fractions of the sample which come from said biological liquid (4) and which are present in each of said analysis cavities (109, 110 or 111), and
- analyzing the result obtained so as to determine the amount of microorganisms or of any other biological component, such as nucleic acids, antibodies, present in the initial biological sample (4).

14. Method as claimed in claim 12, **characterized in that**, once the vacuum has been broken, the isolating fluid is in contact with the biological liquid (4) either in the secondary channels (6) or feed channels (25) or in the analysis cavities (9, 10 and/or 11 or else 29, 30 and/or 31).

15. Method as claimed in either of claims 12 and 14, **characterized in that** the volume of the biological liquid (4) to be analyzed is less than the total volume of all of the analysis cavities (9, 10 and 11 or else 29, 30 and 31).

16. Method as claimed in claim 13, **characterized in that** the volume of the biological liquid (4) to be analyzed is greater than or equal to the total volume of all of the analysis cavities (109, 110 and 111), of all of the fluid circuit (105 to 108 and 112 to 118) and of the overflow (104).

17. Method as claimed in either of claims 13 and 16, **characterized in that** the volume of the biological liquid (4) to be analyzed is greater than or equal to the total volume of all of the analysis cavities (109, 110 and 111) and of all of the fluid circuit (105 to 108 and 112 to 118).

18. Method as claimed in any one of claims 12, 14 or 15, **characterized in that** the ratio of the volume of the biological liquid (4) to be analyzed to the total volume of all of the analysis cavities (9, 10 and 11 or else 29, 30 and 31) is from 1/10 to 9/10, especially from 4/10 to 6/10, and preferably 5/10.

19. Use of an analysis card (1, 21 or 101), as claimed in any one of claims 1 to 11, for counting microorganisms using the technique of the most probable number.
